(19)
**Europäisches Patentamt**
**European Patent Office**
**Office européen des brevets**

(11) **EP 4 591 989 A1**

(12) **EUROPEAN PATENT APPLICATION**
published in accordance with Art. 153(4) EPC

(43) Date of publication:
30.07.2025 Bulletin 2025/31

(21) Application number: 23868027.6

(22) Date of filing: 31.08.2023

(51) International Patent Classification (IPC):
*B03B 5/62* (2006.01)       *B01J 19/00* (2006.01)
*C12M 1/00* (2006.01)       *G01N 1/10* (2006.01)
*G01N 15/00* (2024.01)      *G01N 35/08* (2006.01)
*G01N 37/00* (2006.01)

(52) Cooperative Patent Classification (CPC):
B01J 19/00; B03B 5/62; C12M 1/00; G01N 1/10;
G01N 15/00; G01N 35/08; G01N 37/00

(86) International application number:
PCT/JP2023/031957

(87) International publication number:
WO 2024/062903 (28.03.2024 Gazette 2024/13)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 21.09.2022  JP 2022149952
15.03.2023  JP 2023040661
30.08.2023  JP 2023140211

(71) Applicants:
• CANON KABUSHIKI KAISHA
Tokyo 146-8501 (JP)
• Canon Medical Systems Corporation
Otawara-shi, Tochigi 324-8550 (JP)

(72) Inventors:
• MIKI, Tsutomu
Tokyo 146-8501 (JP)
• KOJIMA, Makoto
Tokyo 146-8501 (JP)
• HOSHIHIRA, Takamitsu
Tokyo 146-8501 (JP)
• HIGUCHI, Yuhi
Tokyo 146-8501 (JP)
• NAGOYA, Toshimitsu
Tokyo 146-8501 (JP)
• UCHIDA, Kouichi
Tokyo 146-8501 (JP)
• SAKAI, Tetsuya
Tokyo 146-8501 (JP)

(74) Representative: TBK
Bavariaring 4-6
80336 München (DE)

(54) **MICROCHANNEL DEVICE, SEPARATION DEVICE, AND SEPARATION METHOD**

(57)     A micro flow path device includes a flow path revolving along a curve, in which a flow path cross section obtained by cutting the flow path in a direction orthogonal to a direction in which a liquid in which particles are dispersed flows has an asymmetric shape. In the micro flow path device, in the flow path cross section having the asymmetric shape, a flow path cross-sectional area S1 on an inner circumferential side with respect to a line segment LS connecting a point PP having a maximum distance to a lower side (12) on an upper side (11) and the lower side by the shortest distance is larger than a flow path cross-sectional area S2 on an outer circumferential side with respect to the line segment LS.

FIG.4A

**EP 4 591 989 A1**

## Description

### Technical Field

The present invention relates to a micro flow path device, a separation device including the micro flow path device, and the like.

### Background Art

In recent years, in various fields such as engineering, chemistry, and biomedicine, a technology for classifying fine particles dispersed in a liquid and a technology for separating and extracting specific blood cells (corpuscles) from a blood specimen are required. Hitherto, a density gradient centrifugation method in which classification, and separation and extraction are performed using a density difference of a target object is known, but in the density gradient centrifugation method, a large centrifugal force is applied to the target object, and thus, there is a possibility that a state change such as damage occurs in the target object in the course of processing.

Therefore, as a method of performing separation and extraction while suppressing damage to a target object dispersed in a liquid, a method of flowing a liquid in a spiral flow path to classify or separate particles has been developed. For example, in the case of separating and extracting minute particles such as blood cells (corpuscles), a spiral flow path is formed by a flow path called a micro flow path having a small flow path cross-sectional area. In general, the micro flow path refers to a narrow flow path in which an outer edge of a flow path cross section cut in a direction orthogonal to a direction in which a liquid flows is formed by a combination of sides of 1 mm or less. However, it is not necessary to strictly limit the micro flow path to a flow path having a cross section formed by a combination of sides of 1 mm or less.

By using the spiral micro flow path, it is possible to expect device miniaturization and an increase in processing speed (throughput) when separating and extracting minute particles such as blood cells (corpuscles). In addition, for example, even in a use in which a component that comes into contact with a specimen needs to be disposable as in a medical inspection, if a structure of the micro flow path can be simplified, there is a possibility that mass production can be performed at low cost, and high practicability as a disposable inspection device can be expected.

Patent Literature 1 describes that a spiral micro flow path used in a micro flow path device has a trapezoidal cross-sectional shape defined by an inner side in a radial direction of the spiral, an outer side in the radial direction, a bottom side, and an upper side. More specifically, Patent Literature 1 describes a trapezoidal flow path cross section in which the bottom side of the trapezoid is linear, the inner side and the outer side in the radial direction are substantially orthogonal to the bottom side, and the upper side is inclined with respect to the bottom side.

Patent Literature 2 describes a microchip that is not a spiral micro flow path but is configured to move an exposed portion of a flow path of external flow path means to switch the flow path.

Patent Literature 3 describes a control method for controlling a flow of a fluid by gelling a sol-gel transition substance added to the fluid by applying a stimulus to a desired portion on a micro flow path that is not a spiral micro flow path.

### Citation List

### Patent Literature

Patent Literature 1: WO 2014/046621 A
Patent Literature 2: JP 2005-214741 A
Patent Literature 3: JP 2002-163022 A

### Summary of Invention

### Technical Problem

The micro flow path device described in Patent Literature 1 adopts a trapezoidal shape in which a height monotonously changes from the inner side to the outer side in the radial direction of the spiral. As a result, a position of a Dean vortex core can be brought closer to one side in the radial direction of the spiral as compared with a micro flow path having a rectangular cross section with a uniform height.

Patent Literature 1 describes that, if the cross-sectional shape of the flow path is a trapezoid in which the height of the inner side in the radial direction of the spiral is larger than that of the outer side in the radial direction, two Dean vortex cores are unevenly distributed on the inner side in the radial direction, so that the micro flow path suitable for concentration and filtration of particles is obtained. In addition, it is described that, if the cross-sectional shape of the flow path is a trapezoid in which the height of the outer side in the radial direction of the spiral is larger than the height of the inner side in the radial direction of the spiral, two Dean vortex cores are unevenly distributed on the outer side, so that the micro flow path suitable for separating particles by size is obtained.

However, as described in Patent Literature 1, in the micro flow path in which cross-sectional shape is a trapezoid, the inner side and the outer side in the radial direction of the spiral are substantially orthogonal to the bottom side, and the height of the trapezoid monotonously changes from the inner side in the radial direction to the outer side in the radial direction, particle classification performance is not necessarily sufficient.

Therefore, there has been a demand for a micro flow path device having more excellent particle classification performance than the related arts.

**Solution to Problem**

According to one aspect of the present invention, there is provided a micro flow path device including a flow path revolving along a curve, in which a flow path cross section obtained by cutting the flow path in a direction orthogonal to a direction in which a liquid in which particles are dispersed flows has an asymmetric shape, and a flow path cross-sectional area S1 on an inner circumferential side with respect to a line segment LS connecting a point PP having a maximum distance to a lower side on an upper side and the lower side by a shortest distance is larger than a flow path cross-sectional area S2 on an outer circumferential side with respect to the line segment LS.

**Advantageous Effects of Invention**

According to the present invention, particles of different sizes contained in a liquid can be classified with high accuracy using a micro flow path.

Other features and advantages of the present invention will become apparent from the following description with reference to the accompanying drawings. In the accompanying drawings, the same or similar configurations are denoted by the same reference numerals.

**Brief Description of Drawings**

Fig. 1 is a schematic view illustrating a configuration of a separation device according to a first embodiment.
Fig. 2 is a schematic plan view illustrating a schematic configuration of a micro flow path according to the embodiment.
Fig. 3A is a schematic view illustrating a state in which the micro flow path is cut along a plane CL illustrated in Fig. 2.
Fig. 3B is a view illustrating a flow path cross-sectional shape in the first embodiment.
Fig. 4A is a view for describing an area and the like of each portion of a flow path cross section.
Fig. 4B is a view for describing a dimension and the like of each portion of the flow path cross section.
Fig. 5A is a view for describing a flow of a liquid in the micro flow path.
Fig. 5B is a view illustrating a distribution of particles in a flow path cross section of a relatively upstream portion of the spiral flow path.
Fig. 5C is a view illustrating a distribution of particles in a flow path cross section of a relatively downstream portion of the spiral flow path.
Fig. 6A is a diagram illustrating a measurement result of a liquid collected in a container 8B.
Fig. 6B is a diagram illustrating a measurement result of a liquid collected in a container 8A.
Fig. 7 is an external view of a mold for manufacturing a micro flow path device.
Fig. 8 is an external view of a resin molded product which is a component of the micro flow path device.
Fig. 9 is a schematic diagram for describing a method of assembling the micro flow path device.
Fig. 10 is an external view of the completed micro flow path device.
Fig. 11A is a view illustrating a flow path cross-sectional shape in which an outer side 14 is bent as another embodiment.
Fig. 11B is a view illustrating a flow path cross-sectional shape in which an outer side 14 has a curved shape as another embodiment.
Fig. 11C is a view illustrating a flow path cross-sectional shape in which an outer side 14 has a stepped shape as another embodiment.
Fig. 12A is a view illustrating a flow path cross-sectional shape in which only a side of the lower side 12 protrudes toward an outer circumferential side as another embodiment.

Fig. 12B is a view illustrating a flow path cross-sectional shape in which only a portion within a predetermined height range protrudes toward an outer circumferential side as another embodiment.

Fig. 13A is a view illustrating a spiral micro flow path that three-dimensionally revolves as another embodiment.

Fig. 13B is a view illustrating a spiral micro flow path having a constant radius of curvature and three-dimensionally revolves as another embodiment.

Fig. 14 is a view illustrating a rectangular cross-sectional shape of a spiral micro flow path used as Comparative Example.

Fig. 15 is a schematic view illustrating a configuration of a micro flow path system 100 according to a second embodiment.

Fig. 16 is a schematic plan view illustrating a schematic configuration of a micro flow path according to the second embodiment.

Fig. 17 is a view illustrating a flow path cross-sectional shape of the micro flow path.

Fig. 18 is a schematic view for describing a mechanism for separating particles having different particle diameters.

Fig. 19 is a schematic view illustrating a configuration of a micro flow path system 200 according to a third embodiment.

Fig. 20 is a schematic view illustrating a configuration of a micro flow path system 300 according to a fourth embodiment.

Fig. 21 is a schematic view illustrating a configuration of a micro flow path system 400 according to a fifth embodiment.

Fig. 22 is a schematic view illustrating a configuration of a micro flow path system 500 according to a sixth embodiment.

Fig. 23 is a schematic view illustrating a micro flow path system 91 according to a reference embodiment.

Fig. 24 is a diagram illustrating a temporal transition of an actual flow rate in the micro flow path.

**Description of Embodiments**

A micro flow path device, a separation device, and the like according to embodiments of the present invention will be described with reference to the drawings. The embodiments described below are merely examples, and for example, detailed configurations can be appropriately changed and implemented by those skilled in the art without departing from the gist of the present invention.

In the drawings referred to in the following description of the embodiments and examples, elements denoted by the same reference numerals have the same functions unless otherwise specified.

In addition, the drawings may be schematic for convenience of illustration and description, and thus, the shape, size, arrangement, and the like of elements in the drawings may not strictly match those of actual ones.

In the following description, the term "particles" or "fine particles" is used to broadly include solid particles containing an organic material, an inorganic material, or both, and objects that can exist in a state of being dispersed in a liquid, such as blood cells (corpuscles) contained in blood.

[First Embodiment]

(Configuration of Separation Device)

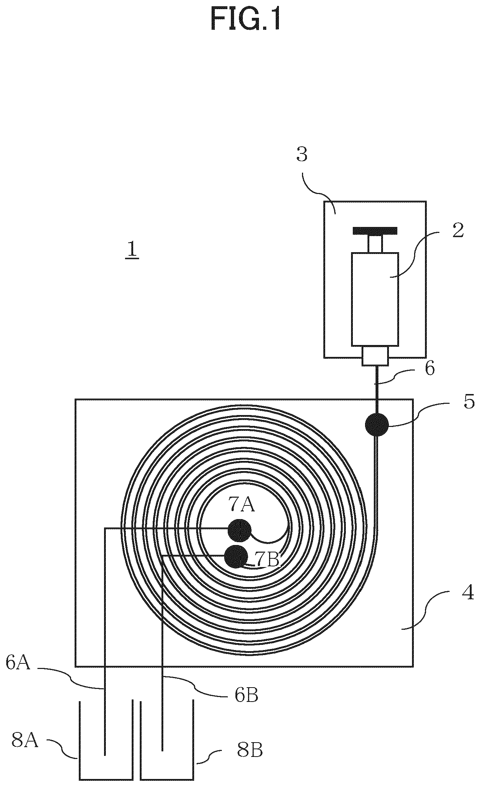

Fig. 1 is a schematic view illustrating a configuration of a separation device according to a first embodiment. A separation device 1 is a device that separates and extracts particles having a large particle diameter and particles having a small particle diameter from particles dispersed in a liquid. The separation device 1 includes a liquid container 2, a pump 3, a micro flow path device 4, a connection portion 5, a tube 6, a tube 6A, a tube 6B, a connection portion 7A, a connection portion 7B, a container 8A, and a container 8B.

The liquid container 2 is a container that holds a liquid (suspension) in which particles having different sizes are dispersed. As the liquid container 2, a syringe (for example, a 10 ml syringe manufactured by Terumo Corporation) can be used, but the liquid container 2 is not limited thereto.

The pump 3 is a pump for pushing out the liquid (suspension) held in the liquid container 2 from the liquid container 2. A syringe pump (for example, YSP-301 manufactured by YMC CO., LTD.) can be used as the pump 3, but the pump 3 is not limited thereto.

The tube 6 is a tube that connects the pump 3 and the connection portion 5. The tube 6 functions as a flow path for guiding the liquid (suspension) pushed out of the liquid container 2 by the pump 3 to the micro flow path device 4. For example, a silicone resin tube having an inner diameter $\varphi$ of 1 mm and an outer diameter $\varphi$ of 3 mm can be used as the tube 6, but the tube 6 is not limited thereto.

The connection portion 5 is a portion that connects the tube 6 and an inlet of a flow path of the micro flow path device 4. It is practically preferable that the connection portion 5 has a mechanism that allows the tube 6 to be easily attached to and detached from the micro flow path device 4.

The micro flow path device 4 is a device capable of separating the particles in the liquid according to a size and taking out the particles separately from the connection portion 7A and the connection portion 7B by allowing the liquid (suspension) introduced via the connection portion 5 to flow along the flow path having a spiral shape. The spiral shape refers to a state along a revolving curve, and is typically a shape similar to a spiral. Details of the micro flow path device 4 will be described below.

The connection portion 7A is a portion that connects the tube 6A and an inner circumferential side of an outlet of the flow path of the micro flow path device 4. The connection portion 7B is a portion that connects the tube 6B and an outer circumferential side of the outlet of the flow path of the micro flow path device 4. It is practically preferable that the connection portion 7A and the connection portion 7B have a mechanism that allows the tube to be easily attached to and detached from the micro flow path device 4.

The container 8A is a container connected to the connection portion 7A via the tube 6A, and stores a liquid containing the particles having a large particle diameter separated by the micro flow path device 4. The container 8B is a container connected to the connection portion 7B via the tube 6B, and stores a liquid containing the particles having a small particle diameter separated by the micro flow path device 4.

(Configuration of Micro Flow Path Device)

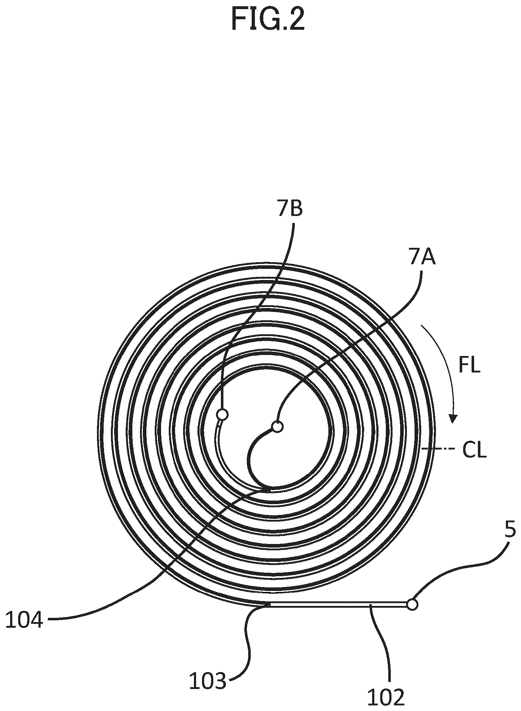

Next, the micro flow path device 4 will be described. Fig. 2 is a schematic plan view illustrating a configuration of a micro flow path included in the micro flow path device 4. Describing from an upstream portion to a downstream portion of the micro flow path, the micro flow path starting from the connection portion 5 is connected to an outer circumferential side end portion 103 of a spiral portion via a straight portion 102 and revolves in the spiral shape to reach an inner circumferential side end portion 104. At the inner circumferential side end portion 104, the micro flow path branches into two branches of an inner circumferential side branch and an outer circumferential side branch, the inner circumferential side branch extends to the connection portion 7A, and the outer circumferential side branch extends to the connection portion 7B. The outer circumferential side end portion 103 which is an upstream end of the spiral portion functions as an injection portion for injecting the liquid, and the inner circumferential side end portion 104 which is a downstream end functions as a branch portion at which the spiral flow path branches into the inner circumferential side branch and the outer circumferential side branch.

For example, a length of the straight portion 102 can be 20 mm, a radius of curvature of a circumference at the outer circumferential side end portion 103 can be 24 mm, the spiral makes eight revolutions such that the radius of curvature decreases by 2 mm for each revolution, and a radius of curvature at the inner circumferential side end portion 104 can be 8 mm. However, this is merely an example, and the configuration of the micro flow path is not limited to this example.

In the micro flow path device 4 according to the present embodiment, a flow path cross section of the spiral portion of the micro flow path has a unique flow path cross-sectional shape. The flow path cross-sectional shape refers to a cross-sectional shape when the flow path is cut along a plane CL orthogonal to FL at an arbitrary position, where FL is a direction in which the liquid flows at an arbitrary position in the micro flow path.

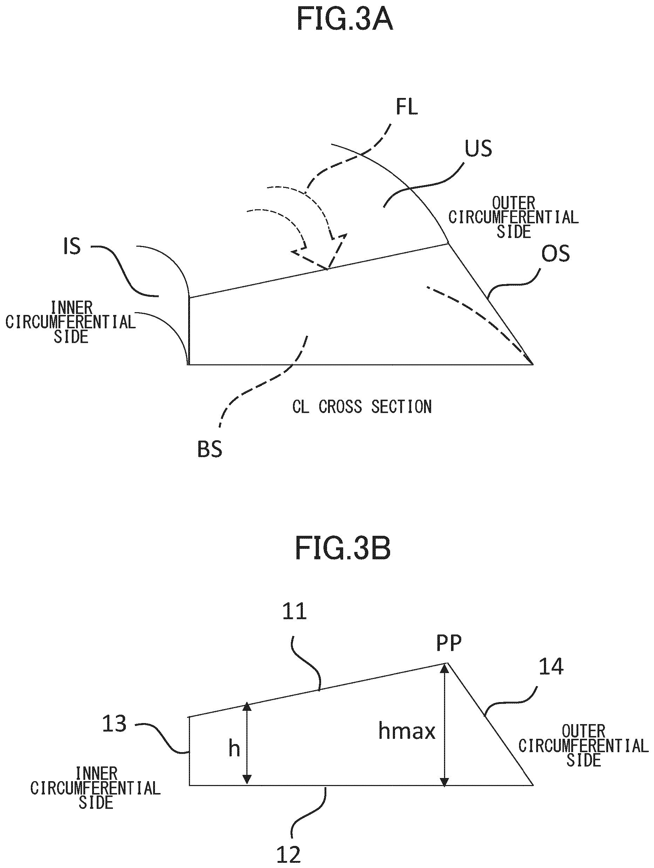

Fig. 3A schematically illustrates a state in which the micro flow path is cut along the plane CL illustrated in Fig. 2. The spiral micro flow path is a tubular flow path defined by an upper surface US, a lower surface BS, an inner circumferential side surface IS, and an outer circumferential side surface OS. An interval between the upper surface US and the lower surface BS of the flow path gradually increases from the inner circumferential side toward the outer circumferential side, and the outer circumferential side surface OS is inclined with respect to the inner circumferential side surface IS. As a result, the flow path cross-sectional shape is an asymmetric quadrangle.

The characteristic flow path cross-sectional shape in the present embodiment will be described in detail with reference to Figs. 3B to 4B. In the present embodiment, the flow path cross section has an asymmetric quadrangular shape including an upper side 11 corresponding to the upper surface US of the tubular path, a lower side 12 corresponding to the lower surface BS of the tubular path, an inner side 13 corresponding to the inner circumferential side surface IS of the tubular path, and an outer side 14 corresponding to the outer circumferential side surface OS of the tubular path.

As illustrated in Fig. 3B, when a distance between the upper side 11 and the lower side 12 is h, h gradually increases from the inner circumferential side toward the outer circumferential side, and the distance to the lower side 12 is maximum (hmax) at a point PP which is an outer circumferential side end portion of the upper side 11. That is, the point PP at which the distance to the lower side 12 is maximum is a boundary point between the upper side 11 and the outer side 14. In other words, a boundary between the upper surface US and the outer circumferential side surface OS of the micro flow path is at a position where a distance between the upper surface US and the lower surface BS becomes maximum. In the illustrated example, a portion of the upper side 11 from a connection point with the inner side 13 of the flow path cross section to the point PP is formed by a straight line.

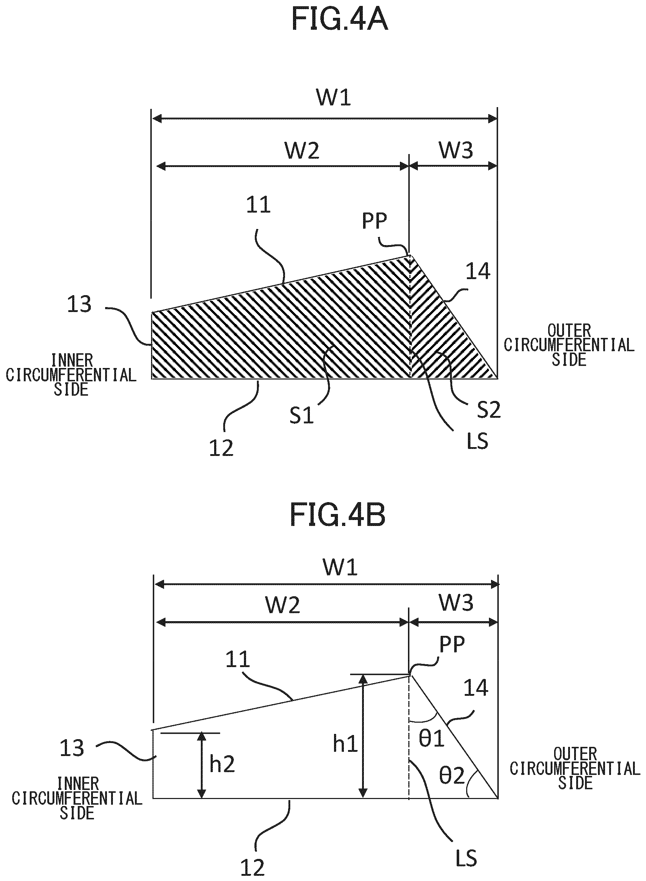

Fig. 4A illustrates a width of each portion of the flow path cross-sectional shape. A width of the spiral micro flow path in plan view (Fig. 2) is W1, a width of the upper side 11 from the inner circumferential side end portion to the point PP is W2, and a width of the outer side 14 from the point PP to the outer circumferential side end portion is W3. Note that W2 may be paraphrased as the width of the upper side 11 in plan view, and W3 may be paraphrased as the width of the outer side 14 in plan view.

As illustrated in the following Formula (1), W2 is larger than W3.

$$W2 > W3 \cdots (1)$$

The formula means that the point PP at which the distance to the lower side 12 is maximum on the upper side 11 (that is, the point PP which is the outer circumferential side end portion of the upper side 11) is positioned on the outer circumferential side with respect to the center in a width direction in plan view of the micro flow path.

In this example, since the inner side 13 has a width of 0 in plan view, W1 = W2 + W3, but even in a case where the inner side 13 is inclined to have a width in plan view, the point PP (that is, the outer circumferential side end portion of the upper side 11) is still positioned on the outer circumferential side with respect to the center of the micro flow path in the width direction.

In addition, when a point at which the distance to the lower side 12 on the upper side 11 is maximum is defined as the point PP, a line segment connecting the point PP and the lower side 12 by the shortest distance is defined as a line segment LS as illustrated in the drawing. When a flow path cross-sectional area on the inner circumferential side is denoted by S1 and an area on the outer circumferential side is denoted by S2 with the line segment LS as a boundary, S1 is larger than S2 as illustrated in the following Formula (2).

$$S1 > S2 \cdots (2)$$

In addition, as illustrated in Fig. 4B, when a length of the line segment LS is h1 and an angle formed by the line segment LS and the outer side 14 is θ1, a relationship of the following Formula (3) is established.

$$10° < θ1 \leq \arctan(W2/h1) \cdots (3)$$

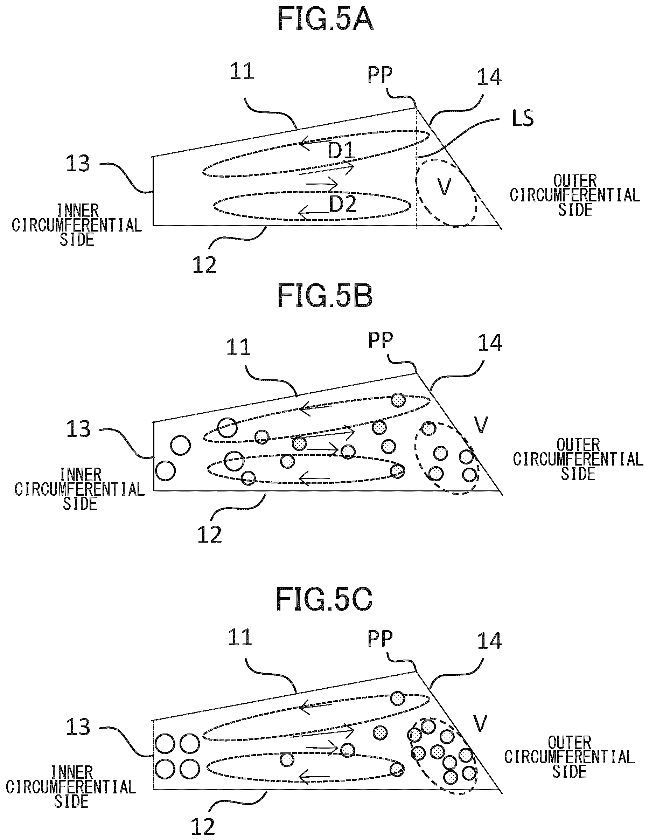

In the spiral micro flow path having the characteristic cross-sectional shape described above, a Dean flow D1 and a Dean flow D2 can be formed in the micro flow path as indicated by dotted lines in Fig. 5A.

As described above, in the present embodiment, the point PP at which the distance between the upper side 11 and the lower side 12 is maximum is disposed on the outer circumferential side with respect to the center of the micro flow path in the width direction. Therefore, centers of vortices of the Dean flow D1 and the Dean flow D2 are shifted to the outer circumferential side from the center in the width direction, and a gap between the Dean flow D1 and the Dean flow D2 spreads vertically from the inner circumferential side toward the outer circumferential side. In addition, a portion protruding from the line segment LS connecting the point PP and the lower side 12 by the shortest distance toward the outer circumferential side in plan view can function as a region V in which the particles having a small diameter and guided by a combination of a Dean drag force and an inertial lift force are stably unevenly distributed on the outer circumferential side. If the flow path cross section is continuously observed while moving with the flow, the particles having a small particle diameter appear to gradually stagnate in the region V on the outer circumferential side, and thus the region V can also be referred to as a stagnation region V.

For example, in the case of separating corpuscles having a large particle diameter (for example, white corpuscles) and corpuscles having a small particle diameter (for example, red corpuscles) from blood cells contained in blood, excellent separation performance can be exhibited by forming a micro flow path having a cross-sectional shape satisfying the above Formulas (1) to (3). When a distribution of the corpuscles in the blood is observed in the spiral micro flow path, a state change occurs from the state illustrated in Fig. 5B to the state illustrated in Fig. 5C as the corpuscles circulate in the spiral flow path from upstream to downstream, and the corpuscles having a small particle diameter are stably unevenly distributed in the region V. When the corpuscles having a small particle diameter can stably stay in the region V (stagnation region V), it is possible to suppress migration of the corpuscles to the inner circumferential side along the Dean vortex again. As a result, it is possible to reduce migration and mixing of the corpuscles having a small particle diameter into the corpuscles having a large particle diameter which are unevenly distributed on the inner circumferential side in the micro flow path. Therefore, the corpuscles (for example, the white corpuscles and the red corpuscles) separated with high purity can be taken out from the connection portion 7A and the connection portion 7B of the micro flow path device 4.

(Manufacturing Method of Micro Flow Path Device)

Next, a manufacturing method of the micro flow path device 4 will be described. As a manufacturing method suitable for mass production, a method of manufacturing a resin micro flow path by injection molding using a metal mold will be described. However, this is an example, and the manufacturing may be performed by a manufacturing method other than the above manufacturing method.

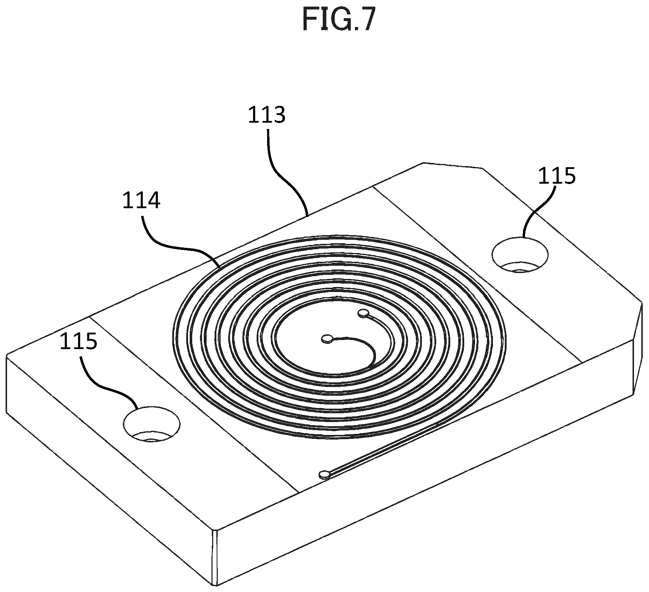

First, the metal mold for transfer-molding the spiral flow path is manufactured. For example, NAK80 is used as a base material, and a mold is produced by cutting using a vertical machining center V56 of Makino Inc. Fig. 7 illustrates an appearance of the produced mold. A mold 113 includes a molding surface 114 for transfer-molding the spiral micro flow path, and an attachment hole 115 used for attachment to an injection molding machine.

Subsequently, injection molding is performed using the mold 113. For example, the mold is set in an injection molding machine SE-75DU manufactured by Sumitomo Heavy Industries, Ltd., and injection molding is performed using a resin material Panlite LV-2250Z manufactured by TEIJIN LIMITED. as a molding material.

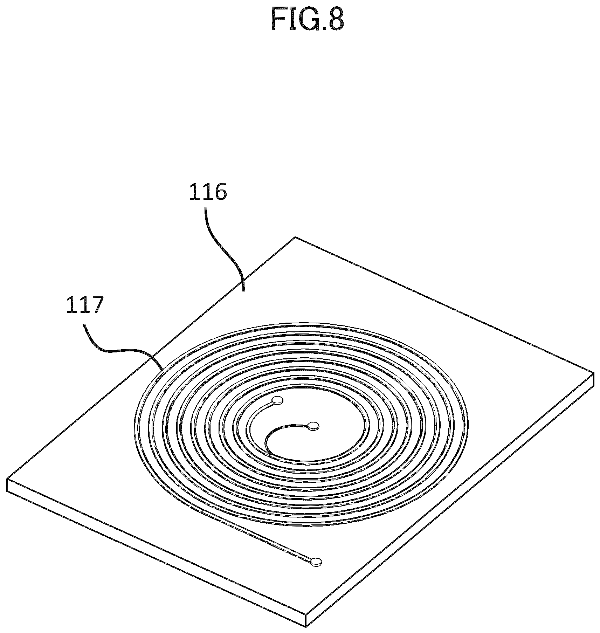

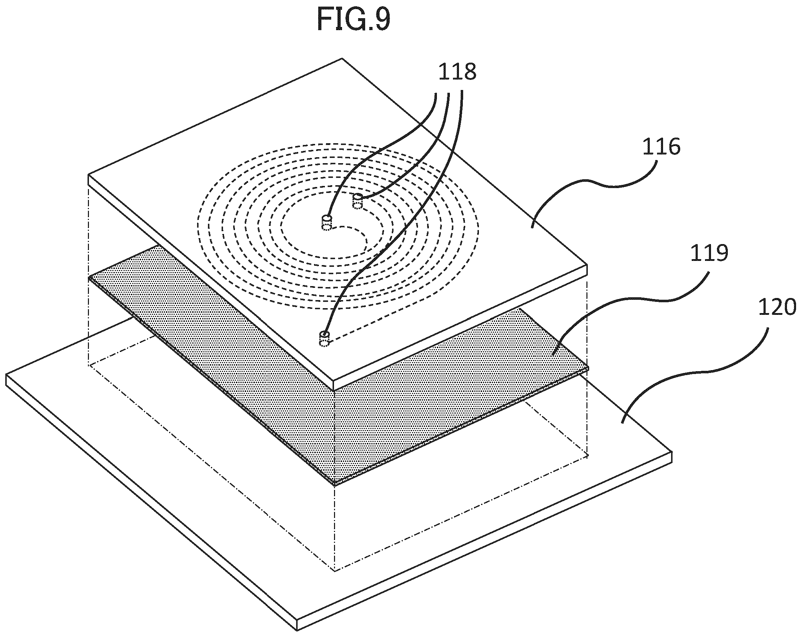

Fig. 8 illustrates an appearance of a resin molded product 116, and a groove 117 that is a part of the spiral micro flow path is formed on an upper surface of the resin molded product 116. Next, holes 118 (see Fig. 9) penetrating to a back surface of the resin molded product 116 are formed at three positions at end portions of the groove 117 in order to provide the connection portion 5, the connection portion 7A, and the connection portion 7B (see Fig. 2). A size of the hole 118 may be, for example, $\Phi 1.5$ mm, but is not limited thereto.

Next, as illustrated in Fig. 9, the resin molded product 116 is positioned at a position where the surface on which the groove 117 is formed faces a substrate 120, and the resin molded product 116 and the substrate 120 are bonded using an adhesive 119. For example, as the adhesive 119, an adhesive transfer tape #9969 for a micro flow path diagnosis chip manufactured by 3M Japan Limited can be used, and as the substrate 120, a model number PCTSH of a polycarbonate resin sheet manufactured by MISUMI Corporation can be used.

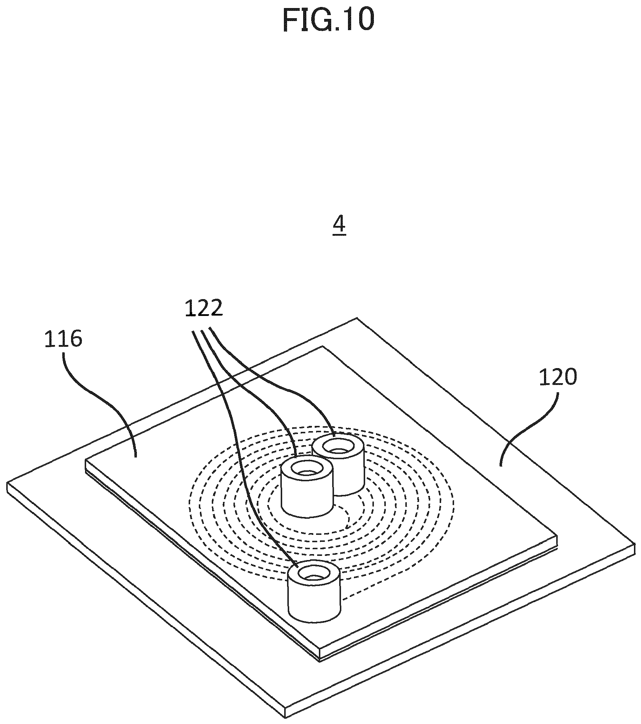

Next, the micro flow path device 4 in which the connection portion 5, the connection portion 7A, and the connection portion 7B are provided is completed by bonding pipette ports 122 to the three holes of the resin molded product 116 as illustrated in Fig. 10.

[Example]

A specific example according to the first embodiment will be described. In Example, the spiral portion of the micro flow path (see Fig. 4B) had a cross-sectional shape in which W1 = 675 $\mu$m, W2 = 600 $\mu$m, h1 = 150 $\mu$m, h2 = 100 $\mu$m, and $\theta 2$ = 60°. As for the shape of the micro flow path in plan view (see Fig. 2), the length of the straight portion 102 was 20 mm, the radius of curvature of the circumference at the outer circumferential side end portion 103 was 24 mm, the spiral made eight revolutions such that the radius of curvature decreased by 2 mm for each revolution, and the radius of curvature at the inner circumferential side end portion 104 was 8 mm.

In order to verify performance in classifying the fine particles dispersed in the liquid, two types of samples were prepared, and classification performance (separation performance according to the particle size) was evaluated.

(Sample 1) Suspension of Resin Fine Particles

Beads made of polystyrene and having a particle diameter of 5 $\mu$m (particle standard 4205A) and beads having a particle diameter of 10 $\mu$m (particle standard 4210A) were dispersed in pure water to prepare a suspension. Components of the suspension were adjusted such that the total amount of the suspension was 5 mL, a proportion of number of 5 $\mu$m particles was 99.85%, and a proportion of number of 10 $\mu$m particles was 0.15%.

(Sample 2) Diluted Blood

Blood collected from a donor was diluted with physiological saline (Dulbecco's Phosphate-Buffered Saline manufactured by NACALAI TESQUE, INC., without Ca and Mg) 1000 times. The total amount of the diluted blood was 5 mL.

(Classification Performance for Sample 1)

5 mL of the suspension containing the beads of Sample 1 was filled in the liquid container 2, and the liquid was fed to the micro flow path device 4 at a flow rate of 1.2 [mL/min] using the pump 3. How many particles of each size were contained in the liquid separated and collected in the container 8A and the container 8B via the micro flow path device 4 was measured. Specifically, the particles contained in the liquid collected in each container were measured using a particle size distribution measuring instrument FPIA-3000 manufactured by Sysmex Corporation.

Here, a collection rate and a removal rate are adopted as indices for evaluating the classification performance (the separation performance according to the particle size) of the separation device 1. The collection rate represents a ratio of the number of 10 $\mu$m beads collected in the container 8A to the total number of 10 $\mu$m beads collected in the container 8A and the container 8B. The removal rate represents a ratio of the number of 5 $\mu$m beads collected in the container 8B to the total number of 5 μm beads collected in the container 8A and the container 8B. The "collection rate" and the "removal rate" are terms used for convenience assuming a use in which 10 μm beads are extracted from the suspension and used, and may be referred to by different names as long as the terms are used as numerical values indicating the performance in separating 10 μm beads and 5 μm beads contained in the suspension.

In Example, the removal rate for Sample 1 was 75.7%, and the collection rate for Sample 1 was 99.8%. It can be seen from the value of the removal rate that about 70% or more of 5 μm beads having a small size were discharged from the connection portion 7B on the outer circumferential side of the spiral micro flow path to the container 8B. It can be seen from the value of the collection rate that most of 10 μm beads having a large size were collected from the connection portion 7A on the inner circumferential side of the spiral micro flow path to the container 8A.

(Classification Performance for Sample 2)

Sample 2 was fed to the micro flow path device 4 in the same procedure as for Sample 1, and the liquid was collected in the container 8A and the container 8B. Then, how many cells of each size were contained in the liquid separated and collected in the container 8A and the container 8B was analyzed. A flow cytometer SA3800 manufactured by Sony Corporation was used, and a cell count number was 50,000. A ratio of lymphocytes contained in each liquid separated and collected in the container 8A and the container 8B was measured in order to grasp a separation effect for corpuscles as fine particles.

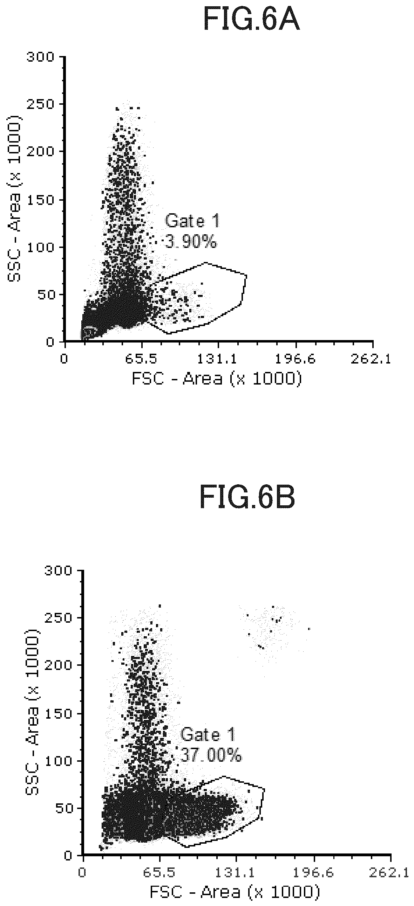

Fig. 6A illustrates measurement results of the liquid collected in the container 8B from the outlet of the spiral micro flow path on the outer circumferential side, and Fig. 6B illustrates measurement results of the liquid collected in the container 8A from the outlet of the spiral micro flow path on the inner circumferential side. A horizontal axis FSC represents an intensity of forward scattered light of the flow cytometer and is proportional to a size of the blood cell. A vertical axis SSC represents an intensity of side scattered light of the flow cytometer and represents an internal complexity of the blood cell. A point cloud in a gate (Gate 1) illustrated in the drawing corresponds to a point cloud of the lymphocytes. As illustrated in Fig. 6A, the cells contained in the liquid collected from the outer circumferential side of the spiral micro flow path were cells having a low FSC, that is, many cells having a small size, and a proportion of the lymphocytes was about 3.9%. On the other hand, as illustrated in Fig. 6B, in the cells contained in the liquid collected from the inner circumferential side of the spiral micro flow path, a cell population having a small size decreased, and the proportion of the lymphocytes was as high as 37.0%. According to such a result, it was confirmed that cells having a small size were easily discharged to the outside of the spiral micro flow path, and cells having a large size were easily collected from the inside.

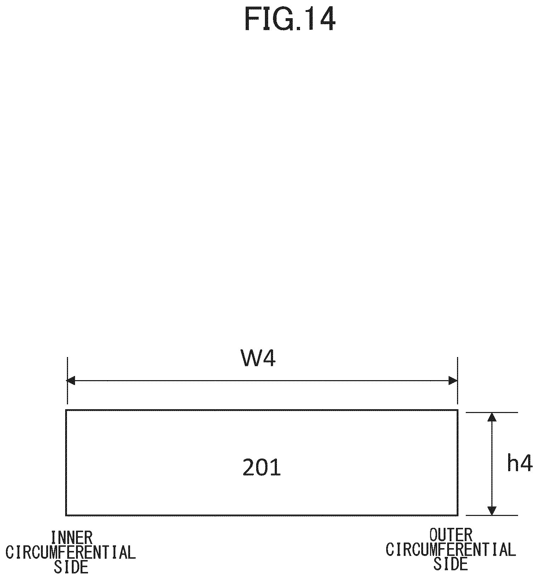

Here, comparison with a case of using a micro flow path device in which a cross-sectional shape of a micro flow path having a spiral shape is rectangular is described as Comparative Example. Fig. 14 illustrates a rectangular cross-sectional shape 201 of the spiral micro flow path used as Comparative Example. A width W4 of the flow path was 675 μm, and a height h4 was 105 μm. The spiral shape in plan view was the same as in Example. A flow path cross-sectional area of Comparative Example is different from that of Example, and in a case where the same flow rate is set, a flow velocity is different, and appropriate comparison cannot be performed. Therefore, the flow rate of Comparative Example is set such that an average flow velocity in the flow path is the same as that of Example.

Blood collected from two donors was diluted 1000 times as described above, and caused to flow through the micro flow path devices of Example and Comparative Example, and the liquid collected from the outlet of the spiral micro flow path on the inner circumferential side was analyzed by the flow cytometer described above. The proportion of the lymphocytes was extracted as described above, and comparison results are illustrated in Table 1. As shown in Table 1, a result indicating that the extracted proportion of the lymphocytes was higher in Example than in Comparative Example in which the flow path cross-sectional shape was rectangular was obtained for the corpuscles of any donor. That is, it was confirmed that Example was suitable for separating blood cells from blood according to a size and extracting blood cells of a desired type.

[Table 1]

| | Extraction Rate of Cells of Predetermined Size [%] | |
|---|---|---|
| | Comparative example | Example |
| Donor A | 7.5 | 30 |
| Donor B | 25.6 | 46.5 |

[Modified Example]

Note that the present invention is not limited to the embodiment and example described above, and many modifications can be made within the technical idea of the present invention. The spiral micro flow path of the micro flow path device according to the present invention may have the following characteristics, and is not limited to the above-described embodiment and example.

(1) The spiral portion has an asymmetric flow path cross section.
(2) The flow path cross-sectional area S1 on the inner circumferential side with respect to the line segment LS connecting the point PP having the maximum distance to the lower side on the upper side and the lower side by the shortest distance is larger than the flow path cross-sectional area S2 on the outer circumferential side with respect to the line segment LS.

As the flow path cross section has an asymmetric shape in which the stagnation region having a smaller cross-sectional area than an inner circumferential side cross-sectional area is formed on the outer circumferential side with respect to the line segment LS in the width direction, a center position of the Dean vortex can be positioned on the outer circumferential side with respect to the center, unlike a case where the flow path cross section is rectangular. As a result, it is possible to prevent the particles having a small particle diameter that have moved to the outer circumferential side from migrating to the inner circumferential side again along the Dean vortex. In other words, mixing of the particles having a small particle diameter due to migration into an aggregate of the particles having a large particle diameter unevenly distributed on the inner circumferential side in the micro flow path due to the Dean flow is reduced. Therefore, the particles can be separated and taken out with higher purity as compared with a micro flow path device having a trapezoidal flow path cross section as described in WO 2014/046621 A.

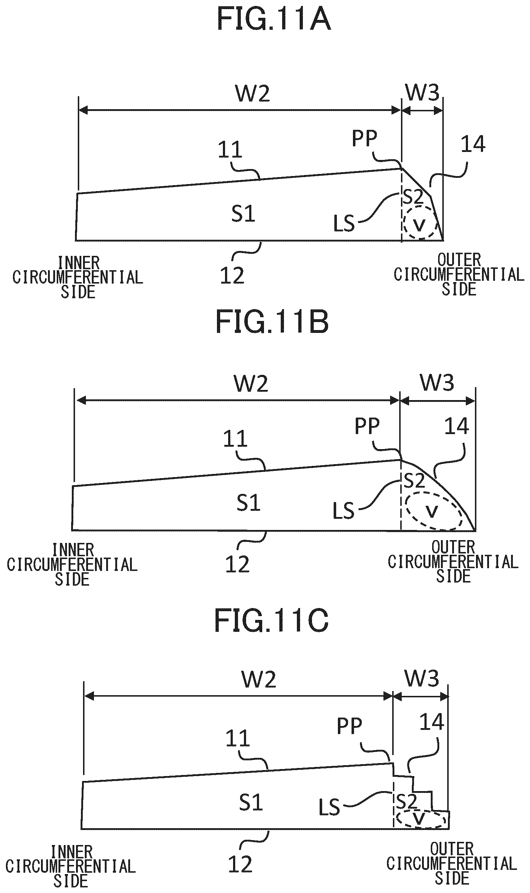

In the example of Fig. 4B described above, the outer side 14 of the flow path cross section is a line segment along one straight line. However, for example, as illustrated in Fig. 11A, the outer side 14 may be a bent outer side 14 in which a plurality of line segments having different inclinations with respect to the lower side 12 are connected. Alternatively, a part of or the entire outer side 14 may have a curved shape as illustrated in Fig. 11B, or the outer side 14 may have a stepped shape as illustrated in Fig. 11C. By setting the shape of the outer side 14 using any one of the shapes or a combination thereof according to an aspect of the particles to be separated and extracted, it is possible to adjust a shape of the Dean flow in a flow path space and a position, shape, and size of the stagnation region, and to achieve high classification performance.

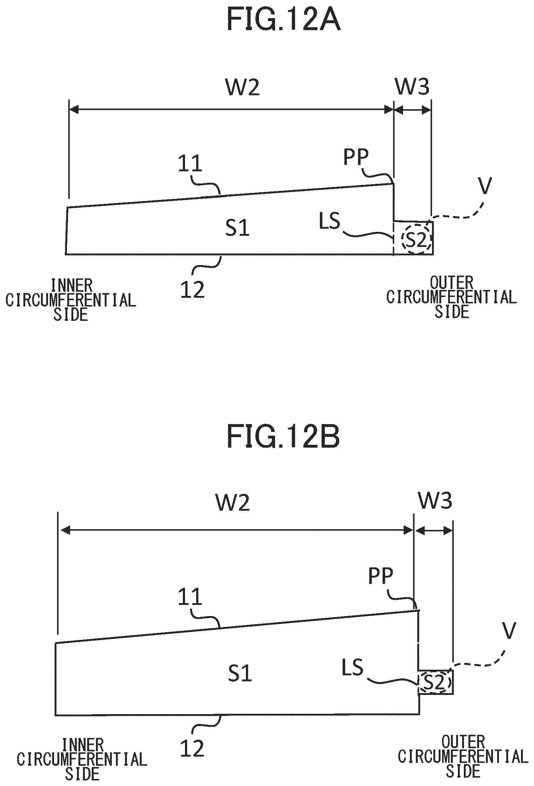

In addition, in the examples of Figs. 4B and 11A to 11C, the region V (stagnation region) for stably and unevenly distributing the particles having a small diameter has a shape in which the outer circumferential side protrudes from the upper side 11 toward the lower side 12, but the region V may have other shapes. For example, as illustrated in Figs. 12A and 12B, in order to form the region V (stagnation region), a protruding portion in which only a portion having a height within a predetermined range protrudes toward the outer circumferential side may be provided on the outer side 14.

In the example of Figs. 4B and 11A to 11C, an inner circumference side and the point PP are connected by one straight line. However, a curve or a wavy line may be used as long as the distance between the point PP and the lower side is maximum.

In addition, for example, in the example of Fig. 2, the spiral micro flow path has a structure in which the liquid is injected from the outer circumferential side end portion 103 and the spiral micro flow path branches into two branches of the inner circumferential side branch and the outer circumferential side branch at the inner circumferential side end portion 104 to separate and collect the liquid, but the spiral micro flow path is not limited thereto. That is, the liquid may be injected from the inner circumferential side end portion of the spiral micro flow path, the spiral micro flow path may branch into two branches of the inner circumferential side branch and the outer circumferential side branch at the outer circumferential side end portion to separate and collect the liquid.

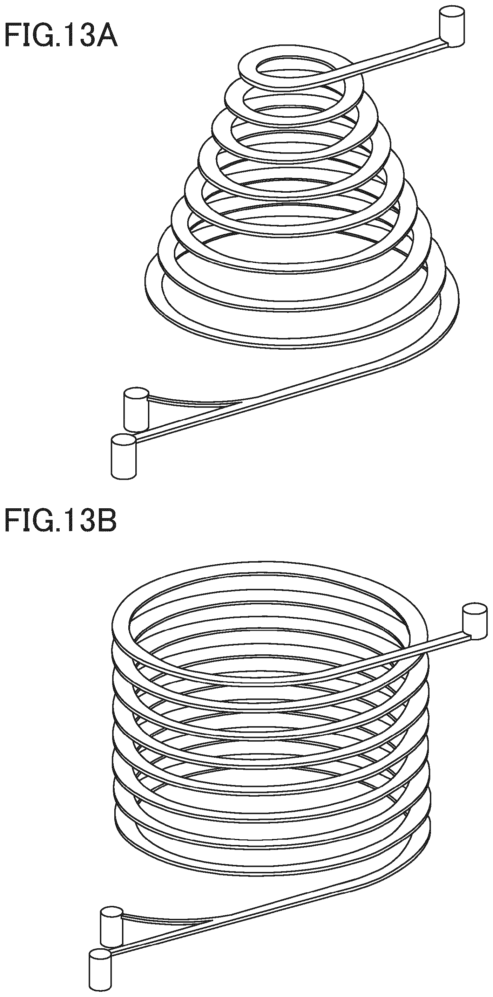

In addition, as illustrated in Fig. 8, in the above-described embodiment, the spiral micro flow path has a structure in which the spiral revolves in a two-dimensional plane, but as schematically illustrated in Fig. 13A, the spiral may revolve in a three-dimensional space. In a case where the spiral three-dimensionally revolves, a spiral structure that revolves with a constant radius of curvature as illustrated in Fig. 13B may be adopted.

In addition, a target object to be handled by the micro flow path device or the separation device according to the present invention is not limited to the suspension containing the polystyrene particles or blood, and various liquids containing various particles having different sizes can be handled as the target object.

As described above, according to the present invention, it is possible to implement a micro flow path device having excellent particle classification performance by having the characteristic flow path cross-sectional shape.

Next, a specific example of a method performed in a reference embodiment will be described in order to facilitate understanding of second to sixth embodiments of the present invention. Examples of the method performed in the reference embodiment include a method of separating cells of different sizes by using a micro flow path, for example, a method of separating blood cells (hereinafter, referred to as corpuscles) according to a size. For example, red corpuscles and white corpuscles are separated from the corpuscles, and the extracted white corpuscles are used for biomedical research and clinical research. In order to obtain a useful research sample, it is required to separate and extract the white corpuscles from a liquid containing the corpuscles with high purity. Even for a purpose other than research, it is still required to separate and extract target corpuscles with high purity.

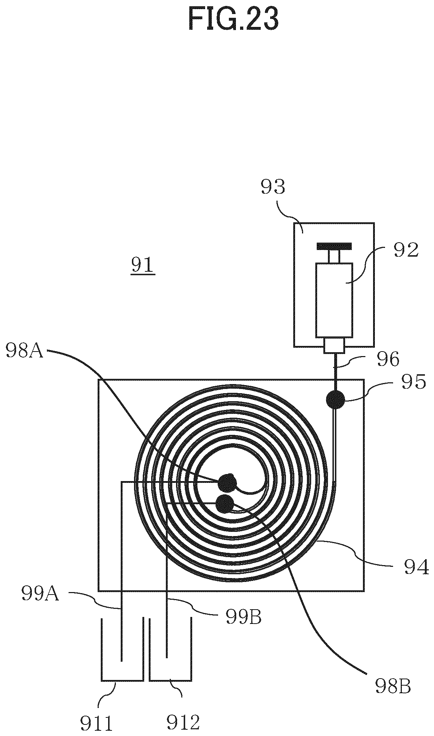

For example, a micro flow path system 91 according to the reference embodiment illustrated in Fig. 23 includes a spiral flow path 94 which is a spiral micro flow path, and can separate the corpuscles according to a difference in size in the spiral flow path 94.

Here, an operation of the micro flow path system 91 according to the reference embodiment will be briefly described. Blood diluted with physiological saline or the like is filled in a syringe 92, and the syringe 92 is attached to a syringe pump 93. The syringe 92 is connected to an injection port 95 of the spiral flow path 94 by a tube 96. The other end of the spiral flow path 94 branches into two ports, an inner discharge port 98A provided on an inner side of the spiral and an outer discharge port 98B provided on an outer side of the spiral. The inner discharge port 98A is connected to a first collection container 911 via a tube 99A, and the outer discharge port 98B is connected to a second collection container 912 via a tube 99B.

When the syringe 92 is pushed out by the syringe pump 93 so as to reach a flow rate suitable for separating the corpuscles in the spiral flow path 94, the diluted blood in the syringe 92 flows into the spiral flow path 94. Although a mechanism will be described below, in a cross section of the spiral flow path, corpuscles having a large size such as the white corpuscles mainly flow on the inner side of the spiral flow path 94 in a radial direction, and corpuscles having a small size such as the red corpuscles and platelets mainly flow on the outer side of the spiral flow path 94 in the radial direction. Therefore, a cell suspension containing a large amount of white corpuscles is discharged to the inner discharge port 98A of the spiral flow path 94, and a cell suspension containing a large amount of red corpuscles and platelets is discharged to the outer discharge port 98B.

The discharged cell suspensions from the respective discharge ports are collected in the first collection container 911 and the second collection container 912 described above. Here, target cells to be separated and extracted with high purity are the white corpuscles, and the white corpuscles are collected in the first collection container 911. In addition, the red corpuscles and the platelets are considered to be unnecessary cells, and the unnecessary cells are discharged to the second collection container 912. As described above, it is possible to separate and collect the corpuscles with a simple system configuration by using the micro flow path system 91.

However, the present inventor has found that when the above-described operation is performed, a large amount of unnecessary red corpuscles and the like are taken into the first collection container 911 that collects the white corpuscles. Details will be described below. Before starting the operation of separating the corpuscles described above, the spiral flow path 94 is not filled with a fluid but is empty. When the cell suspension suddenly flows in a state where the spiral flow path 94 is empty, air in the flow path stagnates in the inner discharge port 98A, the outer discharge port 98B, or the like, and appropriate liquid feeding or separation cannot be performed in some cases. In order to prevent such a problem, physiological saline or the like not containing the corpuscles is caused to flow into the spiral flow path 94 in advance to expel the air in the spiral flow path before the cell suspension flows into the spiral flow path 94.

At this time, for example, in a case where an inevitable minute foreign matter or the like has entered the spiral flow path 94 at the time of manufacturing the spiral flow path 94, there is a possibility that the foreign matter is stored in the first collection container 911 for collecting the target white corpuscles together with the physiological saline or the like.

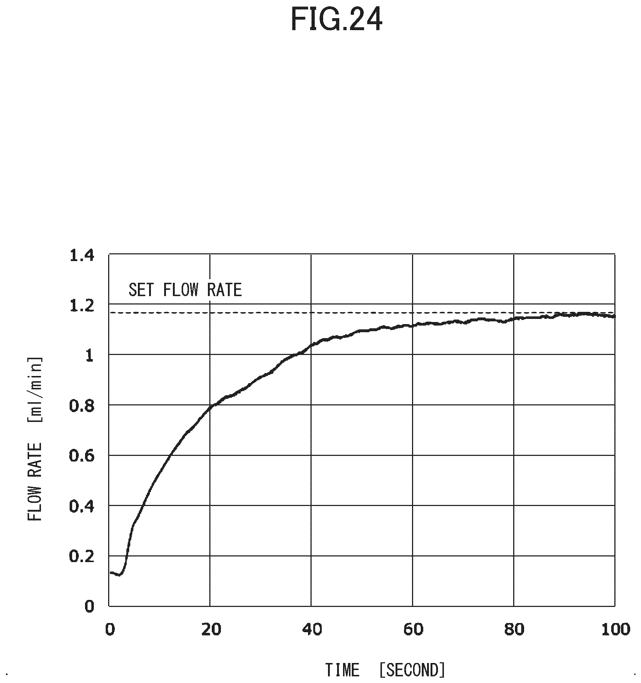

Once the air in the spiral flow path 94 is eliminated, the cell suspension is fed to the spiral flow path 94 from the syringe 92 filled with the cell suspension by the syringe pump 93 at a set flow rate suitable for separating the corpuscles. Fig. 24 illustrates a time transition of an actual flow rate, and it can be seen that a certain amount of time (about 100 seconds in the example of Fig. 24) is required until the flow rate in the spiral flow path 94 reaches the set flow rate suitable for separating the corpuscles.

Since desired cell separation cannot be implemented in the spiral flow path 94 at the set flow rate or less, the unintended red corpuscles and the like are discharged to the first collection container 911 that collects the white corpuscles until the set flow rate is reached. Therefore, the purity of the white corpuscles in the liquid taken into the first collection container 911 decreases.

As described above, during a period from when the air in the spiral flow path 94 is removed and to when the flow rate reaches the set flow rate after the start of feeding of the cell suspension, the liquid is discharged from the inner discharge port 98A of the spiral flow path 94 to the first collection container 911 that collects the white corpuscles as the target object, which is disadvantageous. As a method for preventing such a problem, it is conceivable to provide means for switching the flow path such that the liquid discharged from the inner discharge port 98A is not stored in the first collection container 911 when the cell suspension does not flow at a predetermined flow rate.

Patent Literature 2 described above describes the microchip configured to move the exposed portion of the flow path of the external flow path means to switch the flow path, and it is conceivable to apply the microchip to switching of the flow path in the spiral micro flow path. However, in the method of Patent Literature 2, the flow path is also formed in a lid that covers the flow path, and a liquid feeding line is switched by rotating the lid. However, in this method, it is difficult to move the lid in which the flow path is formed in a sealed state without liquid leakage. Furthermore, a drive mechanism or the like for moving the lid is required, and there is a possibility that the device becomes complicated.

Patent Literature 3 described above describes the method of adding the sol-gel transition substance to the fluid and gelling the fluid at a portion of the flow path where it is not desirable that the fluid flows to block the flow. It is conceivable to apply the method of Patent Literature 3 to switching of the flow path in the spiral micro flow path, but in this method, there is a possibility that physical properties of the fluid are changed and desired separation cannot be performed, or cells are affected. Therefore, the embodiments of the present invention that can solve the problem will be described below.

[Second Embodiment]

(Configuration of Micro Flow Path System 100)

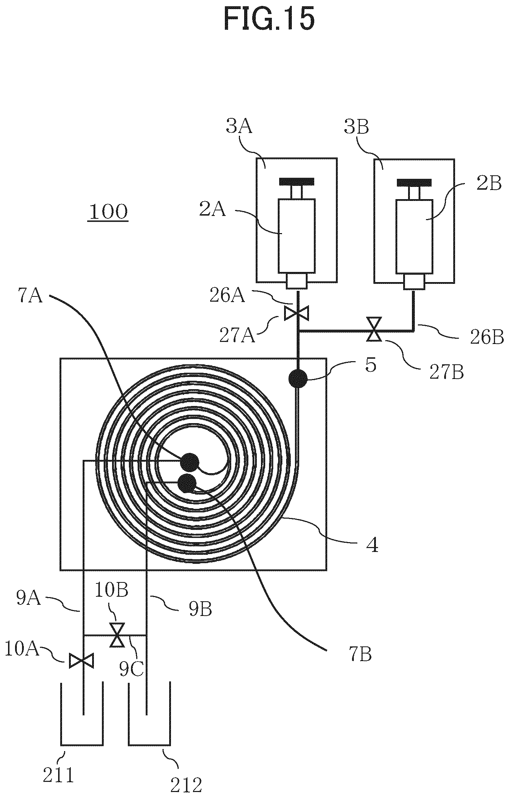

Fig. 15 is a schematic view illustrating a configuration of a micro flow path system 100 according to the present embodiment. The micro flow path system 100 includes a syringe 2A, a syringe 2B, a syringe pump 3A, a syringe pump 3B, a micro flow path device 4 including a spiral flow path, a first container 211, a second container 212, and a control unit CNT.

The control unit CNT is a computer for controlling an operation of each portion of the micro flow path system 100, and internally includes a central processing unit (CPU), a read only memory (ROM), a random access memory (RAM), an input/output (I/O) port, and the like. The ROM stores an operation program of the micro flow path system 100. A program for executing various types of processing of a separation method according to the present embodiment may be stored in the ROM similarly to other operation programs, but may be loaded from the outside into the RAM via a network. Alternatively, the program may be loaded into the RAM via a computer-readable recording medium in which the program is recorded.

The syringe 2A is connected to a connection portion 5 which is an injection port of the spiral flow path included in the micro flow path device 4 via a tube 26A, and the syringe 2B is connected to the connection portion 5 via a tube 26B. The syringe 2A and the syringe pump 3A form a first supply portion capable of supplying a liquid in which particles are dispersed to the micro flow path. The syringe 2B and the syringe pump 3B form a second supply portion capable of supplying a liquid in which the particles are not dispersed to the micro flow path.

A pinch valve 27A is provided on the tube 26A, and a pinch valve 27B is provided on the tube 26B. As operations of the pinch valve 27A and the pinch valve 27B are controllable by the control unit CNT, it is possible to automatically control from which of the syringe 2A and the syringe 2B the liquid is to be fed to the connection portion 5 which is the injection port of the spiral flow path included in the micro flow path device 4.

The spiral flow path included in the micro flow path device 4 branches at a downstream end into two branches of an inner circumferential side branch including a connection portion 7A, which is an inner discharge port, and an outer circumferential side branch including a connection portion 7B which is an outer discharge port. The connection portion 7A as the inner discharge port is connected to a tube 9A, and the connection portion 7B as the outer discharge port is connected to a tube 9B. A pinch valve 10A (first opening/closing portion) is provided in the middle of the tube 9A (first tube). The connection portion 7A as the inner discharge port is connected to the first container 211 via the tube 9A. The connection portion 7B as the outer discharge port is connected to the second container 212 via the tube 9B (second tube). The tube 9A and the tube 9B are connected via a tube 9C (third tube), and a pinch valve 10B (second opening/closing portion) is provided in the middle of the tube 9C. As operations of the pinch valve 10A and the pinch valve 10B are controllable by the control unit CNT, it is possible to automatically control which one of the first container 211 and the second container 212 the liquid discharged from the connection portion 7A as the inner discharge port flows to. On the other hand, since the connection portion 7B is directly connected to the second container 212 by the tube 9B, the liquid discharged from the connection portion 7B as the outer discharge port naturally flows into the second container 212.

In other words, the micro flow path system 100 includes a first flow path that can connect and disconnect the inner circumferential side branch and the first container 211, and a second flow path that connects the outer circumferential side branch and the second container 212 and can connect and disconnect the inner circumferential side branch and the second container.

The micro flow path system 100 has a function of separating corpuscles contained in diluted blood into white corpuscles and (red corpuscles and platelets). Here, for convenience, the white corpuscles as target cells to be separated and extracted with high purity are referred to as necessary cells, and the red corpuscles/platelets are referred to as unnecessary cells. As described below, a liquid containing the high-purity necessary cells separated by the spiral flow path included in the micro flow path device 4 is stored (collected) in the first container 211, and a liquid containing the necessary cells with low purity or a liquid containing the unnecessary cells and impurities is discharged to the second container 212.

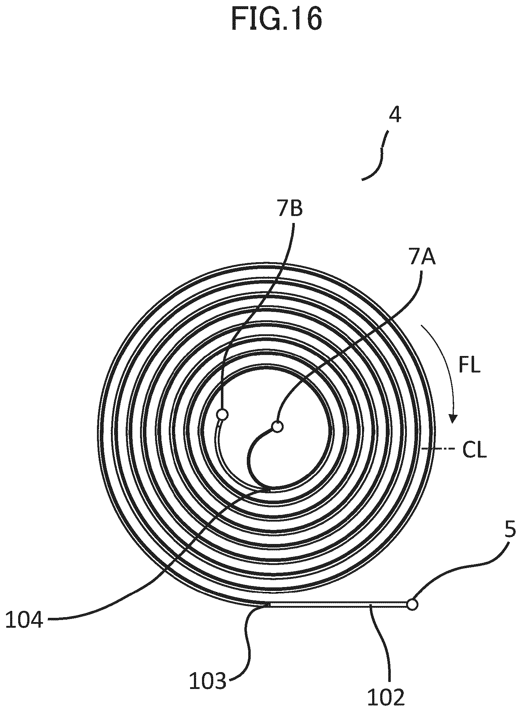

The spiral flow path included in the micro flow path device 4 will be described with reference to Figs. 16 to 18. Fig. 16 is a schematic plan view illustrating a configuration of the spiral flow path included in the micro flow path device 4. Describing from an upstream portion to a downstream portion of the spiral flow path, the micro flow path starting from the connection portion 5, which is the injection port, is connected to an outer circumferential side end portion 103 of a spiral portion via a straight portion 102 and revolves in the spiral shape to reach an inner circumferential side end portion 104. At the inner circumferential side end portion 104, the micro flow path branches into two branches of the inner circumferential side branch and the outer circumferential side branch, the inner circumferential side branch extends to the connection portion 7A as the inner discharge port, and the outer circumferential side branch extends to the connection portion 7B as the outer discharge port. The outer circumferential side end portion 103 which is an upstream end of the spiral portion functions as an injection portion for injecting the liquid into the spiral portion, and the inner circumferential side end portion 104 which is a downstream end functions as a branch portion at which the spiral flow path branches into the inner circumferential side branch and the outer circumferential side branch.

For example, a length of the straight portion 102 can be 20 mm, a radius of curvature of a circumference at the outer circumferential side end portion 103 can be 24 mm, the spiral makes eight revolutions such that the radius of curvature decreases by 2 mm for each revolution, and a radius of curvature at the inner circumferential side end portion 104 can be 8 mm. However, this is merely an example, and the configuration of the micro flow path is not limited to this example.

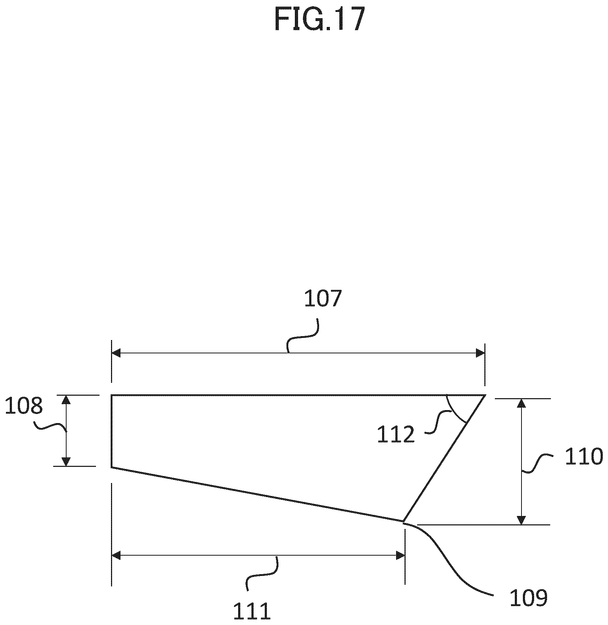

Fig. 17 is a view illustrating a flow path cross-sectional shape of the spiral flow path included in the micro flow path device 4. That is, the flow path cross-sectional shape refers to a cross-sectional shape when the flow path is cut along a plane CL orthogonal to FL at an arbitrary position, where FL is a direction in which the liquid flows at an arbitrary position in the micro flow path as illustrated in Fig. 16. For example, a width 107 of the flow path is 675 $\mu$m, a height 108 on an inner circumferential side of the spiral is 80 $\mu$m, and a height 110 of a highest point 109 on an outer circumferential side is 130 $\mu$m. In addition, a distance 111 from the inner circumferential side end portion of the spiral to the point 109 can be 600 $\mu$m, and an angle 112 of the outer circumferential side end portion of the flow path can be 30°.

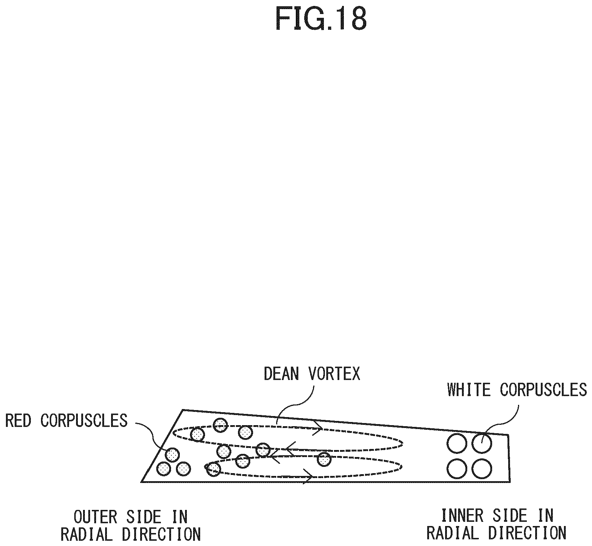

A mechanism in which the spiral flow path included in the micro flow path device 4 separates the corpuscles into the white corpuscles and the other corpuscles (the red corpuscles and the platelets) will be briefly described with reference to Fig. 18. As illustrated in Fig. 18, when a cell suspension is caused to flow through a set flow path in the spiral flow path, a secondary flow is generated in a radial direction of the spiral flow path. The secondary flow is called a Dean vortex. The small-sized red corpuscles and the platelets move outward in the radial direction of the flow path by the Dean vortex. On the other hand, the large-sized white corpuscles move inward in the radial direction of the flow path by an inertial lift force (not illustrated). Therefore, as the red corpuscles flow outward in the radial direction of the flow path and the white corpuscles easily flow inward in the radial direction, the white corpuscles and the other corpuscles (the red corpuscles and the platelets) can be separated by the two branched discharge ports as illustrated in Fig. 16.

As a specific example of each constituent element of the micro flow path system 100, for example, a syringe having a volume of 10 mL manufactured by Terumo Corporation can be used as the syringe 2A and the syringe 2B. For example, YSP-301 manufactured by YMC Co., LTD. can be used as the syringe pump 3A and the syringe pump 3B. For example, a silicone resin tube having an inner diameter $\varphi$ of 1 mm and an outer diameter $\varphi$ of 3 mm can be used as the tube 26A, the tube 26B, the tube 9A, the tube 9B, and the tube 9C. For example, PS-1015NC manufactured by Takasago Electric, Inc. can be used as the pinch valve 27A, the pinch valve 27B, the pinch valve 10A, and the pinch valve 10B.

The syringe 2A is filled with diluted blood obtained by diluting whole blood 500 times with physiological saline (for example, Dulbecco's Phosphate-Buffered Saline manufactured by NACALAI TESQUE, INC., without Ca and Mg). A dilution rate is not limited to 500 times, but if the dilution rate is excessively low, a corpuscle separation mechanism described below becomes difficult to act, and thus, the dilution rate is preferably 100 times or more. Alternatively, a cell suspension obtained by collecting the white corpuscles captured by a filter with physiological saline or the like after roughly removing the red corpuscles with the filter or the like without diluting the whole blood may be filled in the syringe 2A. The syringe 2B is filled with physiological saline (for example, Dulbecco's Phosphate-Buffered Saline manufactured by NACALAI TESQUE, INC., without Ca and Mg) containing no cells.

Note that the specific examples of the constituent elements described above are merely examples, and the present embodiment is not limited thereto.

(Operation of Micro Flow Path System 100)

Next, an operation of the micro flow path system 100 will be described.

First, a priming operation is performed. That is, the control unit CNT closes the pinch valve 27A and opens the pinch valve 27B to drive the syringe pump 3B. As a result, the physiological saline filled in the syringe 2B is injected into the spiral flow path included in the micro flow path device 4, and the spiral flow path is filled with the physiological saline. The priming operation is performed to expel air in the flow path from the flow path. This is because if the air remains in the flow path, when the cell suspension is subsequently caused to flow into the flow path, the flow of the cells is disturbed by the air, and the cells cannot be separated with high accuracy. In the present embodiment, for example, an amount of the physiological saline at the time of priming is set to 5 [mL], and a flow rate at the time of liquid feeding is 1.0 [mL/min], but the present invention is not limited thereto.

In the present embodiment, during the priming operation, the control unit CNT closes the pinch valve 10A and opens the pinch valve 10B. By doing so, even in a case where there is a minute foreign matter or the like in the flow path and the foreign matter flows out from the connection portion 7A as the inner discharge port, the foreign matter does not flow into the first container 211 for collecting the necessary cells, and the foreign matter is discharged to the second container 212. In addition, since the foreign matter is discharged to the second container 212 in a case where the foreign matter flows out of the connection portion 7B as the outer discharge port, the foreign matter does not flow into the first container 211 for collecting the necessary cells. In the priming operation, it can be said that a first process in which the first flow path does not connect the first container to both the inner circumferential side branch and the outer circumferential side branch, and the second flow path connects both the inner circumferential side branch and the outer circumferential side branch to the second container is performed.

When the priming operation is completed, a corpuscle separation operation is started. First, the control unit CNT opens the pinch valve 27A and closes the pinch valve 27B. Then, the syringe pump 3A is driven to feed the diluted blood in the syringe 2A to the spiral flow path included in the micro flow path device 4 at a set flow rate. For example, 1.2 [mL/min] is set as the flow rate suitable for corpuscle separation, but the flow rate is not limited to this example.

Meanwhile, as illustrated in Fig. 24, even in a case where the syringe pump 3A is driven at the set flow rate, a certain amount of time (about 100 seconds in the example of Fig. 24) is required until a flow rate (flow velocity) per unit time in the spiral flow path reaches a predetermined flow rate suitable for separating the corpuscles. Therefore, the spiral flow path cannot exhibit sufficient corpuscle separation performance until the flow rate (flow velocity) of the liquid flowing in the spiral flow path 94 reaches the predetermined flow rate after the driving of the syringe pump 3A is started. Therefore, the red corpuscles and the platelets are mixed in the liquid discharged from the connection portion 7A as the inner discharge port, and the purity of the white corpuscles decreases.

Therefore, in the present embodiment, the control unit CNT closes the pinch valve 10A to prevent the liquid from the connection portion 7A as the inner discharge port from flowing into the first container 211 until the flow rate (flow velocity) per unit time of the liquid flowing in the spiral flow path included in the micro flow path device 4 reaches the predetermined flow rate. At the same time, the control unit CNT opens the pinch valve 10B such that the liquid from the connection portion 7A as the inner discharge port is discharged to the second container 212. It is possible to perform the first process in which the first flow path does not connect both the inner circumferential side branch and the outer circumferential side branch to the first container and the second flow path connects both the inner circumferential side branch and the outer circumferential side branch to the second container, until the flow rate (flow velocity) reaches the predetermined flow rate.

Thereafter, when the flow rate reaches the predetermined flow rate, the control unit CNT opens the pinch valve 10A and closes the pinch valve 10B. With such an operation, only the liquid containing the high-purity necessary cells (for example, the white corpuscles) separated by the spiral flow path included in the micro flow path device 4 is collected in the first container 211, and the liquid containing the unnecessary cells such as the red corpuscles is discharged to the second container 212. In other words, when the flow rate reaches the predetermined flow rate, it is possible to perform a second process in which the first flow path connects the inner circumferential side branch and the first container 211 and the second flow path connects the outer circumferential side branch and the second container 212.

The control unit CNT may control the opening and closing of the pinch valve 10A and the pinch valve 10B based on, for example, a measurement value of a flow rate measurement unit (not illustrated). For example, a flow meter for measuring the flow rate of the diluted blood supplied to the tube 26A may be provided, so that the control unit CNT can monitor the flow rate of the diluted blood flowing in the spiral flow path included in the micro flow path device 4. The control unit CNT can control the opening and closing of the pinch valve 10A and the pinch valve 10B as described above by comparing a measurement result of the flow meter with the set predetermined flow rate.

Alternatively, a predetermined time required for the flow rate of the diluted blood flowing in the spiral flow path included in the micro flow path device 4 to reach the predetermined flow rate from the start of the driving of the syringe pump 3A may be measured in advance and stored in the control unit CNT. The control unit CNT may measure the time with a built-in timer at the same time as the start of the driving of the syringe pump 3A, and switch between the opening and closing of the pinch valve 10A and the pinch valve 10B as described above when the predetermined time has elapsed.

The present embodiment is compared with the method according to the reference embodiment described with reference to Fig. 23. Table 2 illustrates a ratio between the necessary cells (white corpuscles) and the unnecessary cells (red corpuscles) contained in the first container 211 for collecting necessary cells.

[Table 2]

|  | White Corpuscles | Red Corpuscles and Platelets |
| --- | --- | --- |
| Reference Embodiment | 65% | 35% |
| Present Embodiment | 80% | 20% |

As shown in Table 2, according to the present embodiment, a proportion of the white corpuscles contained in the cells collected in the first container was significantly increased as compared with the method according to the reference embodiment. That is, in the present embodiment, particles of different sizes contained in a liquid can be classified with high accuracy using the spiral micro flow path.

[Third Embodiment]

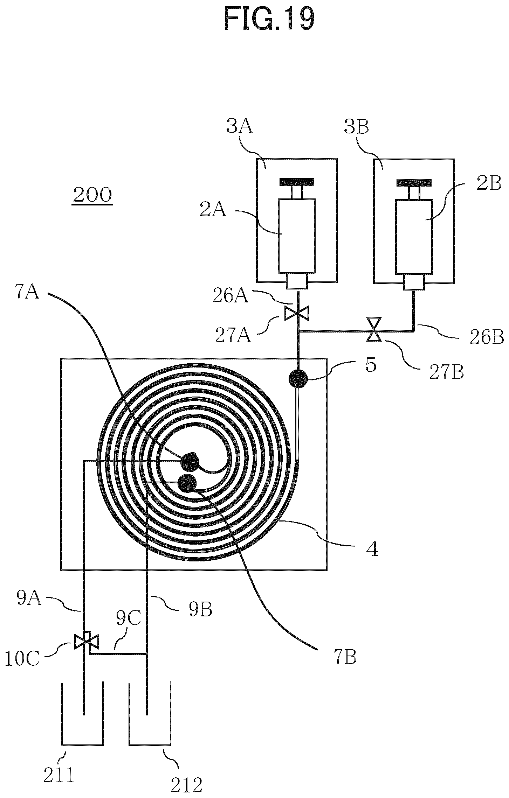

A micro flow path system 200 according to the second embodiment will be described with reference to Fig. 19. In Fig. 19, constituent element common to the second embodiment are denoted by the same reference numerals. A description of matters common to the second embodiment will be simplified or omitted.

The micro flow path system 200 according to the present embodiment also includes a first flow path that can connect and disconnect an inner circumferential side branch and a first container 211, and a second flow path that connects an outer circumferential side branch and a second container 212 and can connect and disconnect the inner circumferential side branch and the second container.

The present embodiment is different from the second embodiment in means for switching the flow path from a spiral flow path included in a micro flow path device 4 to the first container 211 and the second container 212. In the second embodiment, the pinch valve 10A and the pinch valve 10B are provided to switch the flow path. On the other hand, in the present embodiment, a double pinch valve 10C (third opening/closing portion) capable of sandwiching both of tubes 9A and 9C is provided at a portion where branching into the tube 9A (first tube) and the tube 9C (third tube) is made. As the double pinch valve is provided, the tube 9C can be opened at the same time as the tube 9A is closed by one valve. For example, PM-1015W manufactured by Takasago Electric, Inc. can be used as the double pinch valve 10C.

In the present embodiment, a control unit CNT controls the double pinch valve 10C to close the tube 9A and open the tube 9C until a flow rate reaches a set flow rate during a priming operation or a corpuscle separation operation. As a result, it is possible to prevent an unnecessary liquid from flowing into the first container 211 for collecting necessary cells and to discharge the unnecessary liquid to the second container 212.

Then, when conditions for obtaining desired separation performance are satisfied, the control unit CNT controls the double pinch valve 10C to open the tube 9A and close the tube 9C. As a result, a liquid containing the necessary cells with high purity can be taken into the first container 211, and a liquid containing unnecessary cells can be discharged to the second container 212.

In the present embodiment, the number of components can be reduced by using the double pinch valve, and a discharge-side flow path can be easily assembled. In the present embodiment, similarly to the first embodiment, particles of different sizes contained in a liquid can also be classified with high accuracy using the spiral micro flow path.

[Fourth Embodiment]

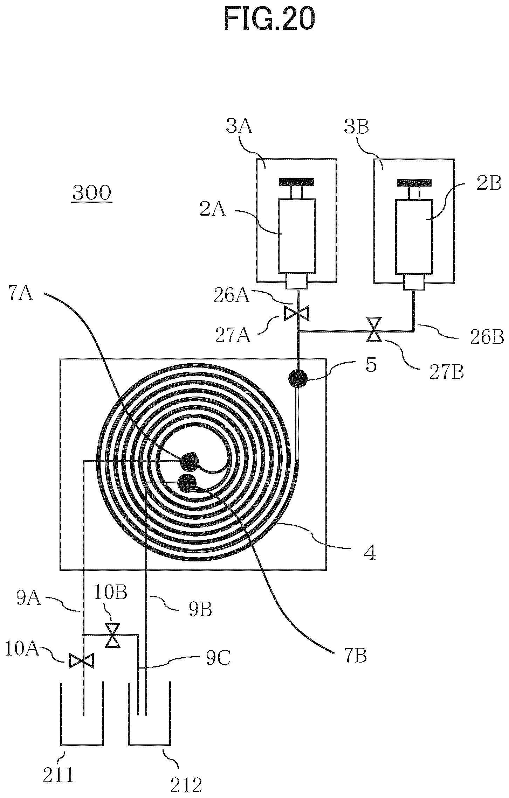

A micro flow path system 300 according to the fourth embodiment will be described with reference to Fig. 20. In Fig. 20, constituent element common to the second embodiment are denoted by the same reference numerals. A description of matters common to the second embodiment will be simplified or omitted.

The micro flow path system 300 according to the present embodiment also includes a first flow path that can connect and disconnect an inner circumferential side branch and a first container 211, and a second flow path that connects an outer circumferential side branch and a second container 212 and can connect and disconnect the inner circumferential side branch and the second container 212.

In the second embodiment, the tube 9C is connected to the tube 9B, and one tube is connected to the second container 212. On the other hand, in the present embodiment, a tube 9C (third tube) and a tube 9B (second tube) are not connected, and the tubes 9C and 9B can independently discharge a liquid to the second container 212.

In the present embodiment, it is possible to reduce one branch portion of the tube (a connection portion between the tubes), it is possible to reduce a pressure loss in a discharge path at the time of liquid feeding, and the discharge path is easily assembled, which is advantageous.

In the present embodiment, similarly to the second embodiment, particles of different sizes contained in a liquid can also be classified with high accuracy using the spiral micro flow path.

[Fifth Embodiment]

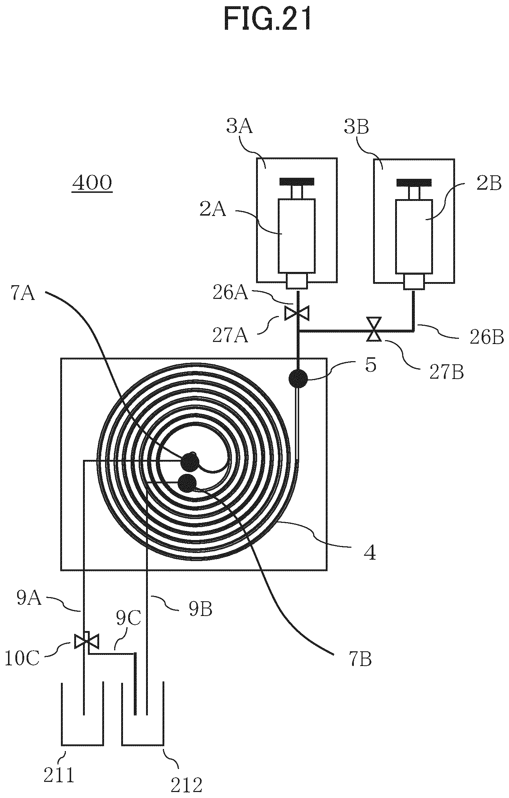

A micro flow path system 400 according to the fifth embodiment will be described with reference to Fig. 21. In Fig. 21, constituent element common to the second embodiment are denoted by the same reference numerals. A description of matters common to the second embodiment will be simplified or omitted.

The micro flow path system 400 according to the present embodiment also includes a first flow path that can connect and disconnect an inner circumferential side branch and a first container 211, and a second flow path that connects an outer circumferential side branch and a second container 212 and can connect and disconnect the inner circumferential side branch and the second container 212.

In the present embodiment, similarly to the fourth embodiment, a tube 9C (third tube) and a tube 9B (second tube) are not connected, and the tubes 9C and 9B can independently discharge a liquid to the second container 212. In the present embodiment, similarly to the third embodiment, a double pinch valve 10C is provided at a portion where branching into a tube 9A (first tube) and the tube 9C is made. As the double pinch valve is provided, the tube 9C can be opened at the same time as the tube 9A is closed by one valve. For example, PM-1015W manufactured by Takasago Electric, Inc. can be used as the double pinch valve 10C.

In the present embodiment, similarly to the second embodiment, particles of different sizes contained in a liquid can also be classified with high accuracy using the spiral micro flow path. In addition, the advantages of the third embodiment and the fourth embodiment can be achieved.

[Sixth Embodiment]

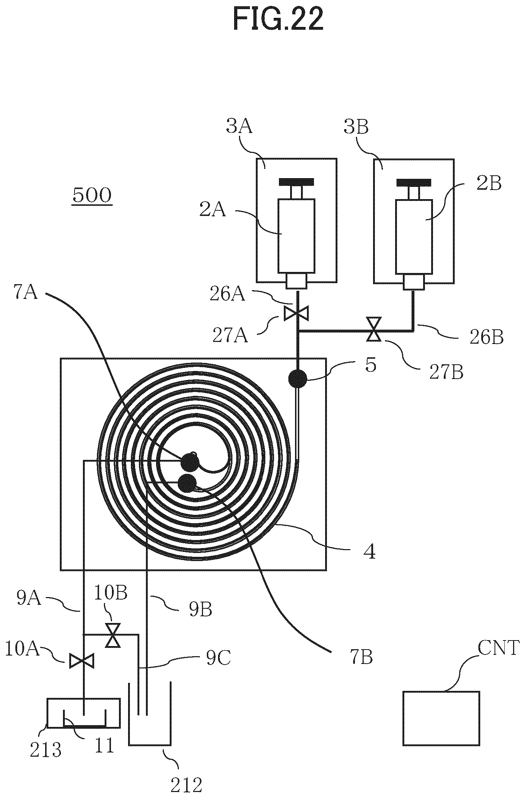

A micro flow path system 500 (separation device) according to the sixth embodiment will be described with reference to Fig. 22. In Fig. 22, constituent element common to the fourth embodiment are denoted by the same reference numerals. A description of matters common to the second or fourth embodiment will be simplified or omitted.

The micro flow path system 500 (separation device) according to the present embodiment also includes a first flow path that can connect and disconnect an inner circumferential side branch and a first container 211, and a second flow path that connects an outer circumferential side branch and a second container 212 and can connect and disconnect the inner circumferential side branch and the second container 212.

A flow path configuration according to the present embodiment is common to that of the fourth embodiment, but is different from that of the fourth embodiment in that the first container 211 for collecting necessary cells is installed in an incubator 213 capable of maintaining a culture environment for cells.

In the present embodiment, similarly to the second embodiment, particles of different sizes contained in a liquid can also be classified with high accuracy using the spiral micro flow path. Furthermore, in the present embodiment, the necessary cells are collected in the first container 211 together with a culture medium, the first container 211 is installed in the incubator 213, and for example, an environment of a temperature of 37°C, a humidity of 90%, and a gas concentration of carbon dioxide ($CO_2$) of 5% is maintained. Therefore, the cells collected in the first container 211 can be cultured as they are. By directly culturing the cells after corpuscle separation, it is possible to increase the number of necessary cells according to the use while reducing a risk of contamination, cell loss when moving the cells to another container, and the like.

[Modified Example]

Note that the present invention is not limited to the embodiments described above, and many modifications can be made within the technical idea of the present invention. For example, all or some of the different embodiments described above may be combined and implemented.

For example, in the above-described embodiments, a case where the necessary particles to be separated with high accuracy are particles having a large particle diameter (for example, the white corpuscles) has been described as an example. Therefore, a flow path configuration in which a liquid discharged from an inner circumferential side discharge port during the separation operation is collected in the first container 211 is adopted, and the liquid is not discharged to the first container 211 during the priming operation or immediately after the injection of the liquid containing the particles. On the other hand, in a case where the necessary particles to be separated with high accuracy are particles having a small particle diameter, a flow path configuration in which a liquid discharged from an outer circumferential side discharge port during the separation operation is collected in the first container 211 is adopted. Then, it is sufficient if the liquid is not discharged to the first container 211 during the priming operation or immediately after the injection of the liquid containing the particles. That is, it is sufficient if one of the inner circumferential side branch and the outer circumferential side branch and the first container 211 are disconnectably connected by the first flow path, and the other of the inner circumferential side branch and the outer circumferential side branch and the second container 212 are always connected by the second flow path according to the particle diameter of the necessary particles.

In addition, the control unit CNT does not have to directly control all the connections and disconnections of the respective flow paths, and some or all of the connections and disconnections may be operable by an operator. In this case, notification means such as a lamp, an audio device, or a display device may be provided in order to enable the operator to appropriately operate an opening/closing device such as a valve, and the control unit CNT may notify the operator of an opening/closing timing via the notification means. Alternatively, the operator may be notified of a timing of switching from the first process to the second process.

In the sixth embodiment, the first container 211 is installed in the incubator 213 in the flow path configuration according to the fourth embodiment, but the first container 211 may also be installed in the incubator 213 in the flow path configurations according to the second, third, and fifth embodiment.

In addition, it is preferable to use the spiral flow path according to the first embodiment as the spiral flow path used in each of the second to sixth embodiments.

In each embodiment, the spiral flow path is used to classify particles having different particle diameters. However, the present invention is not necessarily limited to the spiral flow path as long as the flow path is a micro flow path along a curve having a function of performing separation according to the particle diameter, and micro flow paths having other forms may be used. The spiral flow path is suitably implemented because of an advantage of high throughput.

In addition, a device for controlling the connection and disconnection of the flow path is not limited to the pinch valve or the double pinch valve, and for example, a two-way cock or a tube clamp may be used.

In addition, the particles separated by the micro flow path device are not limited to corpuscles, and may be various particles such as solid particles containing an organic material, an inorganic material, or both, and cells other than corpuscles.

The present specification discloses at least the following items.

[Item 1]

A micro flow path device including:

a flow path revolving along a curve, in which
a flow path cross section obtained by cutting the flow path in a direction orthogonal to a direction in which a liquid in which particles are dispersed flows has an asymmetric shape, and
a flow path cross-sectional area S1 on an inner circumferential side with respect to a line segment LS connecting a point PP having a maximum distance to a lower side on an upper side and the lower side by a shortest distance is larger than a flow path cross-sectional area S2 on an outer circumferential side with respect to the line segment LS.

[Item 2]

The micro flow path device according to Item 1, in which a stagnation region in which particles having a particle diameter smaller than a predetermined particle diameter among the particles contained in the liquid are unevenly distributed is formed in a flow path space on the outer circumferential side with respect to the line segment LS.

[Item 3]

The micro flow path device according to Item 1, in which an outer side of the flow path cross section includes a portion along a straight line.

[Item 4]

The micro flow path device according to Item 3, in which a relationship of $10° < θ1 ≤ \arctan(W2/h1)$, in which h1 represents a length of the line segment LS, and θ1 represents an angle formed by the line segment LS and the straight line, is established.

[Item 5]

The micro flow path device according to Item 1, in which a portion of the upper side from a connection point with an inner side of the flow path cross section to the point PP is formed by a straight line.

[Item 6]

The micro flow path device according to Item 1, in which an outer side of the flow path cross section includes a portion where a plurality of line segments having different inclinations with respect to a lower side are connected.

[Item 7]

The micro flow path device according to Item 2, in which in an outer side of the flow path cross section, only a portion having a height within a predetermined range protrudes toward the outer circumferential side with respect to the line segment LS.

[Item 8]

The micro flow path device according to any one of items 1 to 7, in which the curve revolves in a two-dimensional plane.

[Item 9]

The micro flow path device according to any one of items 1 to 8, in which the curve is a curve whose radius of curvature is smaller in an inner circumference than in an outer circumference.

[Item 10]

A micro flow path device including:

a micro flow path revolving along a curve and provided with a branch portion at which the micro flow path branches into an inner circumferential side branch and an outer circumferential side branch at a downstream end;
a first supply portion configured to supply a liquid in which particles are dispersed to the micro flow path;
a first container configured to store a liquid containing particles having a predetermined particle diameter;
a second container;
a first flow path configured to connect and disconnect any one of the inner circumferential side branch and the outer circumferential side branch and the first container; and
a second flow path configured to connect the other one of the inner circumferential side branch and the outer circumferential side branch and the second container and to connect and disconnect any one of the inner circumferential side branch and the outer circumferential side branch and the second container.

[Item 11]

The micro flow path device according to Item 10, in which

in a case where the first supply portion starts to supply the liquid in which the particles are dispersed to the micro flow path,
after performing a first process in which the first flow path does not connect the first container to both of the inner circumferential side branch and the outer circumferential side branch, and the second flow path connects both the inner circumferential side branch and the outer circumferential side branch to the second container, a second process in which the first flow path connects any one of the inner circumferential side branch and the outer circumferential side branch and the first container, and the second flow path connects the other one of the inner circumferential side branch and the outer circumferential side branch and the second container is performed.

[Item 12]

The micro flow path device according to Item 11, further including a flow rate measurement unit configured to measure a flow rate at which the liquid in which the particles are dispersed is supplied from the first supply portion to the micro flow path,
in which switching from the first process to the second process is made in a case where a measurement result of the flow rate measurement unit reaches a predetermined flow rate.

[Item 13]

The micro flow path device according to Item 11, in which switching from the first process to the second process is made after performing the first process for a predetermined time.

[Item 14]

The micro flow path device according to Item 10, further including a second supply portion configured to supply a liquid in which the particles are not dispersed to the micro flow path,
in which after the liquid in which the particles are not dispersed is supplied from the second supply portion to the micro flow path, the liquid in which the particles are dispersed is supplied from the first supply portion to the micro flow path.

[Item 15]

The micro flow path device according to Item 14, in which while the liquid in which the particles are not dispersed is supplied from the second supply portion to the micro flow path, the first flow path does not connect the first container to both of the inner circumferential side branch and the outer circumferential side branch, and the second flow path connects both the inner circumferential side branch and the outer circumferential side branch and the second container.

[Item 16]

The micro flow path device according to any one of items 11 to 15, in which the micro flow path discharges the liquid containing the particles having the predetermined particle diameter from the inner circumferential side branch, and the first flow path connects the inner circumferential side branch and the first container in the second process.

[Item 17]

The micro flow path device according to Item 10, in which

the first flow path includes a first tube configured to connect one of the inner circumferential side branch and the outer circumferential side branch and the first container, and a first opening/closing portion provided in a middle of the first tube, and
the second flow path includes a second tube configured to connect the other one of the inner circumferential side branch and the outer circumferential side branch and the second container, a third tube configured to connect an upstream portion of the first tube with respect to the first opening/closing portion and the second tube, and a second opening/closing portion provided in a middle of the third tube.

[Item 18]

The micro flow path device according to Item 10, further including:

a first tube configured to connect any one of the inner circumferential side branch and the outer circumferential side branch and the first container;
a second tube configured to connect the other one of the inner circumferential side branch and the outer circumferential side branch and the second container;
a third tube configured to connect the first tube and the second tube; and
a third opening/closing portion configured to control opening and closing of both of the first tube and the third tube.

[Item 19]

The micro flow path device according to Item 10, in which

the first flow path includes a first tube configured to connect one of the inner circumferential side branch and the outer circumferential side branch and the first container, and a first opening/closing portion provided in a middle of the first tube, and
the second flow path includes a second tube configured to connect the other one of the inner circumferential side branch and the outer circumferential side branch and the second container, a third tube configured to connect an upstream portion of the first tube with respect to the first opening/closing portion and the second container, and a second opening/closing portion provided in a middle of the third tube.

[Item 20]

The micro flow path device according to Item 10, further including:

a first tube configured to connect any one of the inner circumferential side branch and the outer circumferential side branch and the first container;
a second tube configured to connect the other one of the inner circumferential side branch and the outer circumferential side branch and the second container;
a third tube configured to connect the first tube and the second container; and
a third opening/closing portion configured to control opening and closing of the first tube and the third tube.

[Item 21]

The micro flow path device according to any one of items 1 to 20, in which the particles are solid particles containing an organic material, an inorganic material, or both, or cells.

[Item 22]

A separation device including:

the micro flow path device according to any one of Items 15 to 20; and
an incubator configured to culture cells, in which
the particles are the cells, and
the first container is installed in the incubator.

[Item 23]

A separation method including:
separating particles having a large particle diameter or particles having a small particle diameter from particles dispersed in a liquid by using the micro flow path device according to any one of Items 1 to 20.

**Industrial Applicability**

The present invention can be widely applied to a device for classifying fine particles dispersed in a liquid and a device for separating and extracting specific blood cells (corpuscles) from a blood specimen in various fields such as engineering, chemistry, and biomedicine.
The present invention is not limited to the above embodiments, and various changes and modifications can be made without departing from the spirit and scope of the present invention. Therefore, in order to make the scope of the present invention public, the following claims are attached.

Reference Signs List

| | |
|---|---|
| 1 | Separation device |
| 2 | Liquid container |
| 3 | Pump |
| 4 | Micro flow path device |
| 5 | Connection portion |
| 6, 6A, 6B | Tube |
| 7A, 7B | Connection portion |
| 8A, 8B | Container |
| 11 | Upper side |
| 12 | Lower side |
| 13 | Inner side |
| 14 | Outer side |
| 102 | Straight portion |
| 103 | Outer circumferential side end portion |
| 104 | Inner circumferential side end portion |
| 113 | Mold |
| 114 | Molding surface |
| 115 | Attachment hole |
| 116 | Resin molded product |
| 117 | Groove |
| 118 | Hole |
| 119 | Adhesive |
| 120 | Substrate |
| 122 | Pipette port |
| 201 | Rectangular cross-sectional shape |
| BS | Lower surface |
| D1, D2 | Dean flow |
| IS | Inner circumferential side surface |
| LS | Line segment |
| OS | Outer circumferential side surface |

US          Upper surface
V           Stagnation region

**Claims**

1. A micro flow path device comprising:

   a flow path revolving along a curve, wherein
   a flow path cross section obtained by cutting the flow path in a direction orthogonal to a direction in which a liquid in which particles are dispersed flows has an asymmetric shape, and
   a flow path cross-sectional area S1 on an inner circumferential side with respect to a line segment LS connecting a point PP having a maximum distance to a lower side on an upper side and the lower side by a shortest distance is larger than a flow path cross-sectional area S2 on an outer circumferential side with respect to the line segment LS.

2. The micro flow path device according to claim 1, wherein a stagnation region in which particles having a particle diameter smaller than a predetermined particle diameter among the particles contained in the liquid are unevenly distributed is formed in a flow path space on the outer circumferential side with respect to the line segment LS.

3. The micro flow path device according to claim 1, wherein an outer side of the flow path cross section includes a portion along a straight line.

4. The micro flow path device according to claim 3, wherein a relationship of $10° < \theta1 \leq \arctan(W2/h1)$, in which h1 represents a length of the line segment LS, and $\theta1$ represents an angle formed by the line segment LS and the straight line, is established.

5. The micro flow path device according to claim 1, wherein a portion of the upper side from a connection point with an inner side of the flow path cross section to the point PP is formed by a straight line.

6. The micro flow path device according to claim 1, wherein an outer side of the flow path cross section includes a portion where a plurality of line segments having different inclinations with respect to the lower side are connected.

7. The micro flow path device according to claim 2, wherein in an outer side of the flow path cross section, only a portion having a height within a predetermined range protrudes toward the outer circumferential side with respect to the line segment LS.

8. The micro flow path device according to any one of claims 1 to 7, wherein the curve revolves in a two-dimensional plane.

9. The micro flow path device according to any one of claims 1 to 8, wherein the curve is a curve whose radius of curvature is smaller in an inner circumference than in an outer circumference.

10. A micro flow path device comprising:

    a micro flow path revolving along a curve and provided with a branch portion at which the micro flow path branches into an inner circumferential side branch and an outer circumferential side branch at a downstream end;
    a first supply portion configured to supply a liquid in which particles are dispersed to the micro flow path;
    a first container configured to store a liquid containing particles having a predetermined particle diameter;
    a second container;
    a first flow path configured to connect and disconnect any one of the inner circumferential side branch and the outer circumferential side branch and the first container; and
    a second flow path configured to connect another one of the inner circumferential side branch and the outer circumferential side branch and the second container and to connect and disconnect any one of the inner circumferential side branch and the outer circumferential side branch and the second container.

11. The micro flow path device according to claim 10, wherein

    in a case where the first supply portion starts to supply the liquid in which the particles are dispersed to the micro flow path,

after performing a first process in which the first flow path does not connect the first container to both of the inner circumferential side branch and the outer circumferential side branch, and the second flow path connects both the inner circumferential side branch and the outer circumferential side branch to the second container, a second process in which the first flow path connects any one of the inner circumferential side branch and the outer circumferential side branch and the first container, and the second flow path connects the other one of the inner circumferential side branch and the outer circumferential side branch and the second container is performed.

12. The micro flow path device according to claim 11, further comprising a flow rate measurement unit configured to measure a flow rate at which the liquid in which the particles are dispersed is supplied from the first supply portion to the micro flow path,

wherein switching from the first process to the second process is made in a case where a measurement result of the flow rate measurement unit reaches a predetermined flow rate.

13. The micro flow path device according to claim 11, wherein switching from the first process to the second process is made after performing the first process for a predetermined time.

14. The micro flow path device according to claim 10, further comprising a second supply portion configured to supply a liquid in which the particles are not dispersed to the micro flow path,

wherein after the liquid in which the particles are not dispersed is supplied from the second supply portion to the micro flow path, the liquid in which the particles are dispersed is supplied from the first supply portion to the micro flow path.

15. The micro flow path device according to claim 14, wherein while the liquid in which the particles are not dispersed is supplied from the second supply portion to the micro flow path, the first flow path does not connect the first container to both of the inner circumferential side branch and the outer circumferential side branch, and the second flow path connects both the inner circumferential side branch and the outer circumferential side branch and the second container.

16. The micro flow path device according to any one of claims 11 to 15, wherein the micro flow path discharges the liquid containing the particles having the predetermined particle diameter from the inner circumferential side branch, and the first flow path connects the inner circumferential side branch and the first container in the second process.

17. The micro flow path device according to claim 10, wherein

the first flow path includes a first tube configured to connect one of the inner circumferential side branch and the outer circumferential side branch and the first container, and a first opening/closing portion provided in a middle of the first tube, and

the second flow path includes a second tube configured to connect the other one of the inner circumferential side branch and the outer circumferential side branch and the second container, a third tube configured to connect an upstream portion of the first tube with respect to the first opening/closing portion and the second tube, and a second opening/closing portion provided in a middle of the third tube.

18. The micro flow path device according to claim 10, further comprising:

a first tube configured to connect any one of the inner circumferential side branch and the outer circumferential side branch and the first container;

a second tube configured to connect the other one of the inner circumferential side branch and the outer circumferential side branch and the second container;

a third tube configured to connect the first tube and the second tube; and

a third opening/closing portion configured to control opening and closing of both of the first tube and the third tube.

19. The micro flow path device according to claim 10, wherein

the first flow path includes a first tube configured to connect one of the inner circumferential side branch and the outer circumferential side branch and the first container, and a first opening/closing portion provided in a middle of the first tube, and

the second flow path includes a second tube configured to connect the other one of the inner circumferential side branch and the outer circumferential side branch and the second container, a third tube configured to connect an upstream portion of the first tube with respect to the first opening/closing portion and the second container, and a second opening/closing portion provided in a middle of the third tube.

20. The micro flow path device according to claim 10, further comprising:

a first tube configured to connect any one of the inner circumferential side branch and the outer circumferential side branch and the first container;
a second tube configured to connect the other one of the inner circumferential side branch and the outer circumferential side branch and the second container;
a third tube configured to connect the first tube and the second container; and
a third opening/closing portion configured to control opening and closing of the first tube and the third tube.

21. The micro flow path device according to any one of claims 1 to 20, wherein the particles are solid particles containing an organic material, an inorganic material, or both, or cells.

22. A separation device comprising:

the micro flow path device according to any one of claims 15 to 20; and
an incubator configured to culture cells, wherein
the particles are the cells, and
the first container is installed in the incubator.

23. A separation method comprising:
separating particles having a large particle diameter or particles having a small particle diameter from particles dispersed in a liquid by using the micro flow path device according to any one of claims 1 to 20.

# FIG.1

# FIG.2

# FIG.3A

FL

US

OUTER
CIRCUMFERENTIAL
SIDE

OS

IS

INNER
CIRCUMFERENTIAL
SIDE

CL CROSS SECTION

BS

# FIG.3B

11

PP

14

13

OUTER
CIRCUMFERENTIAL
SIDE

hmax

h

INNER
CIRCUMFERENTIAL
SIDE

12

# FIG.4A

W1

W2    W3

11

PP

13    14

INNER
CIRCUMFERENTIAL
SIDE

OUTER
CIRCUMFERENTIAL
SIDE

S1    S2

LS

12

# FIG.4B

W1

W2    W3

11    PP

14

13    θ1

h1    OUTER
CIRCUMFERENTIAL
SIDE

h2    θ2

INNER
CIRCUMFERENTIAL
SIDE

12

LS

# FIG.5A

11    PP    14
13    LS
D1
V
INNER
CIRCUMFERENTIAL
SIDE
D2
OUTER
CIRCUMFERENTIAL
SIDE
12

# FIG.5B

11    PP    14
13    V
INNER
CIRCUMFERENTIAL
SIDE
OUTER
CIRCUMFERENTIAL
SIDE
12

# FIG.5C

11    PP    14
13    V
INNER
CIRCUMFERENTIAL
SIDE
OUTER
CIRCUMFERENTIAL
SIDE
12

# FIG.6A

# FIG.6B

FIG.7

# FIG.8

FIG.9

116

118

119

120

# FIG.10

<u>4</u>

# FIG.11A

W2

W3

PP

11

14

S1

S2

LS

V

INNER
CIRCUMFERENTIAL
SIDE

12

OUTER
CIRCUMFERENTIAL
SIDE

# FIG.11B

W2

W3

PP

11

14

S1

S2

LS

V

INNER
CIRCUMFERENTIAL
SIDE

12

OUTER
CIRCUMFERENTIAL
SIDE

# FIG.11C

W2

W3

PP

11

14

S1

LS

S2

V

INNER
CIRCUMFERENTIAL
SIDE

12

OUTER
CIRCUMFERENTIAL
SIDE

# FIG.12A

W2    W3

11    PP    V

S1    LS    S2

INNER
CIRCUMFERENTIAL    12
SIDE

OUTER
CIRCUMFERENTIAL
SIDE

# FIG.12B

W2    W3

11    PP    V

S1    LS    S2

INNER
CIRCUMFERENTIAL    12
SIDE

OUTER
CIRCUMFERENTIAL
SIDE

## FIG.13A

## FIG.13B

# FIG.14

W4

201

h4

INNER
CIRCUMFERENTIAL
SIDE

OUTER
CIRCUMFERENTIAL
SIDE

# FIG.15

# FIG.16

# FIG.17

FIG.18

DEAN VORTEX

WHITE CORPUSCLES

RED CORPUSCLES

OUTER SIDE IN
RADIAL DIRECTION

INNER SIDE IN
RADIAL DIRECTION

# FIG.19

# FIG.20

# FIG.21

# FIG.22

# FIG.23

# FIG.24

## INTERNATIONAL SEARCH REPORT

International application No.

**PCT/JP2023/031957**

### A. CLASSIFICATION OF SUBJECT MATTER

***B03B 5/62*(2006.01)i; *B01J 19/00*(2006.01)i; *C12M 1/00*(2006.01)i; *G01N 1/10*(2006.01)i; *G01N 15/00*(2024.01)i; *G01N 35/08*(2006.01)i; *G01N 37/00*(2006.01)i**

FI: B03B5/62; B01J19/00 N; G01N37/00 101; G01N35/08 A; G01N1/10 A; C12M1/00 Z; G01N15/00 C

According to International Patent Classification (IPC) or to both national classification and IPC

### B. FIELDS SEARCHED

Minimum documentation searched (classification system followed by classification symbols)

B03B5/62; B01J19/00; C12M1/00; G01N1/10; G01N15/00; G01N35/08; G01N37/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2019-515678 A (BIOLIDICS LIMITED) 13 June 2019 (2019-06-13) paragraphs [0055], [0065]-[0077], fig. 4, 9 | 10-11, 13-23 |
| A | | 1-9, 12 |
| A | WO 2016/044555 A1 (MASSACHUSETTS INSTITUTE OF TECHNOLOGY) 24 March 2016 (2016-03-24) entire text, all drawings | 1-9 |
| A | JP 2017-527299 A (MASSACHUSETTS INSTITUTE OF TECHNOLOGY) 21 September 2017 (2017-09-21) entire text, all drawings | 1-9 |
| A | CN 112547145 A (SOUTHEAST UNIVERSITY) 26 March 2021 (2021-03-26) entire text, all drawings | 1-9 |
| A | JP 2015-535728 A (MASSACHUSETTS INSTITUTE OF TECHNOLOGY) 17 December 2015 (2015-12-17) entire text, all drawings | 1-9 |

☐ Further documents are listed in the continuation of Box C.   ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **19 October 2023** | **07 November 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

| International application No. |
|---|
| **PCT/JP2023/031957** |

**Box No. III     Observations where unity of invention is lacking (Continuation of item 3 of first sheet)**

This International Searching Authority found multiple inventions in this international application, as follows:

Document 1: JP 2019-515678 A (BIOLIDICS LIMITED) 13 June 2019 (2019-06-13) paragraphs [0055], [0065]-[0077], fig. 4, 9 & US 2019/0151847 A1 paragraphs [0064], [0074]-[0086], fig. 4, 9 & WO 2017/192098 A1 & EP 3455337 A1 & CN 109072149 A

Claims are classified into the following two inventions.

(Invention 1) Claims 1-9 and 21-23
Claim 1 and claims 9 and 21-23 referring to claim 1 have the special technical feature of a "microfluidic device comprising a flow path rotating along a curve, wherein: a cross-section of the flow path cut along a direction perpendicular to the direction in which the liquid in which the particles are dispersed flows is asymmetric; and the flow path cross-sectional area S1 on the inner circumferential side than the line segment LS connecting the point PP at which the distance between the lower side and the upper side is the maximum and the lower side so that the distance the point PP and the lower side becomes the shortest is larger than the flow path cross-sectional area S2 on the outer circumferential side than the line segment LS," and are thus classified as invention 1.

(Invention 2) Claims 10-23
Claim 10 and claims 11-23 referring to claim 10 share, with claim 1 classified as invention 1, the common technical feature of a "microfluidic device having a flow path that rotates along a curve." However, said technical feature does not make a contribution over the prior art in light of the disclosure of for example, document 1, and thus cannot be said to be a special technical feature.
Also, there are no other identical or corresponding technical features between claim 10 and claims 11-23 referring to claim 10, and claim 1.
In addition, claim 10 and claims 11-23 referring to claim 10 are not dependent on claim 1. In addition, claim 10 and claims and 11-23 referring to claim 10 are not substantially identical to or similarly closely related to any of the claims classified as invention 1.
Thus, claims 10 and claims 11-23 referring to claim 10 cannot be classified as invention 1.
Thus, claims 10 and claims 11-23 referring to claim 10 are classified as invention 2.

1. ☐ As all required additional search fees were timely paid by the applicant, this international search report covers all searchable claims.

2. ☑ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**     ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
Information on patent family members

International application No.

**PCT/JP2023/031957**

| Patent document cited in search report | | | Publication date (day/month/year) | Patent family member(s) | | | Publication date (day/month/year) |
|---|---|---|---|---|---|---|---|
| JP | 2019-515678 | A | 13 June 2019 | US | 2019/0151847 | A1 | |
| | | | | paragraphs [0064], [0074]-[0086], fig. 4, 9 | | | |
| | | | | WO | 2017/192098 | A1 | |
| | | | | EP | 3455337 | A1 | |
| | | | | CN | 109072149 | A | |
| WO | 2016/044555 | A1 | 24 March 2016 | US | 2017/0296732 | A1 | |
| | | | | entire text, all drawings | | | |
| JP | 2017-527299 | A | 21 September 2017 | US | 2017/0292104 | A1 | |
| | | | | entire text, all drawings | | | |
| | | | | WO | 2016/044537 | A1 | |
| | | | | EP | 3194560 | A1 | |
| | | | | CN | 107250341 | A | |
| CN | 112547145 | A | 26 March 2021 | (Family: none) | | | |
| JP | 2015-535728 | A | 17 December 2015 | US | 2015/0238963 | A1 | |
| | | | | entire text, all drawings | | | |
| | | | | WO | 2014/046621 | A1 | |
| | | | | EP | 2897730 | A1 | |
| | | | | EP | 3628401 | A1 | |
| | | | | KR | 10-2015-0061643 | A | |
| | | | | CN | 104797340 | A | |

Form PCT/ISA/210 (patent family annex) (January 2015)

**REFERENCES CITED IN THE DESCRIPTION**

**Patent documents cited in the description**

- WO 2014046621 A
- JP 2005214741 A
- JP 2002163022 A